# EUROPEAN PATENT APPLICATION

(11) **EP 4 667 087 A2**
(43) Date of publication of application: **24.12.2025**
(21) Application number: 25179442.6
(22) Date of filing: 28.05.2025
(51) Int. Cl.: B01D 53/22, B01D 53/30, C10L 3/10

(54) **SYSTEMS AND METHODS FOR BIOGAS UPGRADING USING SWEEP GAS**

(30) Priority: 28.05.2024 US 202463652618 P
(71) Applicant: Haffmans B.V., 5928 PW Venlo (NL)
(72) Inventor: ROODBEEN, J.E., Venlo (NL); BANOS, Konstantinos, Athens (GR); van ESSEN, Machiel, Geldrop (NL); TINOCO, Rodrigo Rivera, Venlo (NL)
(74) Representative: Viering, Jentschura & Partner mbB Patent- und Rechtsanwälte

(57) **Abstract**

A system for separating a gas mixture is provided. The system includes a source of a gas mixture and a membrane separation stage including a gas separation membrane module. The membrane separation stage is in fluid communication with the source of the gas mixture, and the gas separation membrane module is configured to separate the gas mixture into a retentate stream and a permeate stream. The gas separation membrane module also includes a membrane having a permeate side and a retentate side. A sweep stream is provided to the permeate side of the membrane, the sweep stream comprising a portion of the retentate stream.

## Description

### CROSS REFERENCE TO RELATED APPLICATIONS

This application claims priority under 35 U.S.C. § 119 to U.S. Provisional Patent Application Serial No. 63/652,618, filed on May 28, 2024, entitled "SYSTEM AND METHOD FOR BIOGAS UPGRADING USING SWEEP GAS," the entire disclosure of which is incorporated herein by reference.

### BACKGROUND

With global carbon emissions and energy market volatility rising, it is becoming important to transition to renewable energy. Gas separation membranes are commonly used in industry to purify, upgrade, and/or remove a desired component from gas mixtures. Recently, there has been an increased interest in separating methane from biogas or natural gas streams. Methane, especially biomethane, is a desirable product as it can be used in a variety of commercial applications, such as a source of energy for heating, the production of chemicals, or as a fuel, among others. However, efficiently separating methane from carbon dioxide at an industrial scale is a non-trivial task that may require multiple gas separation steps and/or operating at high pressures to achieve the desired purity of the product gas. In particular, operating pressure(s) may impact gas separation because gas permeation is driven by partial pressure differences of the gas components across the gas separation membrane. In fact, several different methods for generating a partial pressure difference across a membrane are known, including increasing the pressure of (compressing) the gas that is fed into a gas separation membrane or by providing a vacuum pump on the permeate side of the membrane. However, the application of these methods in an existing commercial gas separation system may involve additional complexity, including additional steps and/or additional equipment.

There remains a need for improvements to existing commercial gas separation processes to develop more cost-effective and energy-efficient systems for separating methane from carbon dioxide.

### SUMMARY

Various systems and methods for biogas upgrading are provided herein. Each of the systems and methods may separate the biogas (or another provided gas mixture) via a membrane separation apparatus. Each system and method may also utilize a sweep gas drawn from a gas stream that is a component of the gas separation system, such as a feed stream or a retentate stream generated by a membrane separation apparatus, or a gas stream sourced from outside of the gas separation system in order to increase the efficiency of the biogas upgrading. For example, each system and method may utilize a sweep gas provided as an inert gas (e.g., nitrogen gas) that is sourced from a tank or other storage unit in order to increase the efficiency of the biogas upgrading. As an additional example, each system and method may utilize a sweep gas provided from a gas sourced from an apparatus, unit, or system that is downstream of the membrane separation apparatus in order to increase the efficiency of the biogas upgrading. Furthermore, the sweep stream may be internal to or integrated with the membrane separation apparatus or external to the membrane separation apparatus (e.g., not directly coupled to the membrane gas separation apparatus). Surprisingly, the application of a sweep stream may significantly increase membrane CO₂ permeance, while simultaneously increasing membrane CO₂/CH₄ selectivity.

In some aspects, a system for separating a gas mixture is provided in the form of a source of a gas mixture and a membrane separation stage including a gas separation membrane module. The membrane separation stage is in fluid communication with the source of the gas mixture, and the gas separation membrane module is configured to separate the gas mixture into a retentate stream and a permeate stream. The gas separation membrane module also includes a membrane having a permeate side and a retentate side. A sweep stream is provided to the permeate side of the membrane, the sweep stream comprising a portion of the retentate stream.

In some cases, the system further includes a controller in electrical communication with one or more measuring devices configured to measure a parameter of the sweep stream, the retentate stream, and/or the permeate stream.

In some instances, the system further includes one or more process control devices in fluid communication with the sweep stream, the retentate stream, and/or the permeate stream. In some such instances, the controller is configured to send a signal to the one or more process control devices in response to the measurement of the parameter.

In some cases, the parameter is selected from the group consisting of pressure, temperature, flow rate, composition, viscosity, humidity, moisture content, dewpoint, density, and combinations thereof.

In some instances, the controller compares a value of the parameter measured by the one or more measuring devices with a set-point value of the parameter.

In some cases, the system further includes one or more valves in fluid communication with the sweep stream, the retentate stream, and/or the permeate stream. In some such instances, the controller is configured to maintain or adjust a position of the one or more valves in response to the measurement of the parameter.

In some instances, the gas mixture includes methane and carbon dioxide.

In some cases, the system further includes at least one gas transport device with an inlet in fluid communication with the gas mixture and an outlet in fluid communication with the gas separation membrane module.

In some instances, the first membrane separation stage includes a first plurality of membranes having a first total membrane surface area.

In some cases, about 0.1% to about 15% of the retentate stream is provided to the permeate side of the gas separation membrane module as a sweep gas.

In some cases, the gas mixture comprises biogas. In some such cases, the retentate stream is primarily methane (e.g., at least about 80% methane).

In other aspects, a system for separating a gas mixture is provided in the form of a first membrane separation stage in fluid communication with a sweep stream and a second membrane separation stage. The first membrane separation stage includes at least one gas separation membrane module that is configured to separate the gas mixture into a first retentate stream and a first permeate stream. The second membrane separation stage is configured to separate the first permeate stream into a second retentate stream and a second permeate stream. The sweep stream is provided to a permeate side of the first membrane separation stage, and the sweep stream comprises at least a portion of the first retentate stream.

In some instances, the system further includes a first gas transport device with a first inlet in fluid communication with the gas mixture and a first outlet in fluid communication with the first membrane separation stage. In some such instances, a second gas transport device with a second inlet in fluid communication with the first permeate stream and a second outlet in fluid communication with the second membrane separation stage is provided.

In some cases, the first membrane separation stage comprises a first plurality of membranes having a first total membrane surface area.

In some instances, the second membrane separation stage comprises a second plurality of membranes having a second total membrane surface area.

In some cases, the sweep stream comprises at least a portion of the first retentate stream.

In some instances, the sweep stream comprises a gas stream sourced from outside of the system.

In some cases, the gas mixture is provided as a feed gas to the system, and the sweep stream comprises no more than about 8% of the feed gas.

In yet other aspects, a method for separating a gas mixture is provided. The method comprises the steps of (a) providing the gas mixture comprising methane and carbon dioxide, (b) feeding the gas mixture to a membrane separation stage comprising at least one gas separation membrane module configured to separate the gas mixture into a retentate stream and a permeate stream, wherein the at least one gas separation membrane module comprises a membrane having a permeate side and a retentate side, wherein a first pressure of gas on the retentate side is greater than a second pressure of gas on the permeate side, and (c) feeding a sweep stream along the permeate side of the membrane, wherein the sweep stream comprises a portion of the retentate stream.

In other aspects, the method further comprises (d) feeding the first permeate stream to a second membrane separation stage comprising at least one gas separation membrane module configured to separate the first permeate stream into a second retentate stream and a second permeate stream, wherein the at least one gas separation membrane module of the second membrane separation stage comprises a membrane having a permeate side and a retentate side.

In some instances, the method further includes the step of providing a controller in electrical communication with one or more measuring devices configured to measure a parameter of the sweep stream, the first retentate stream, the second retentate stream, the first permeate stream, and/or the second permeate stream.

In other instances, the method further comprises (e) comparing a value of the parameter measured by the one or more measuring devices with a set-point value of the parameter and adjusting one or more valves in fluid communication with at least one of the sweep stream, the retentate stream, and the permeate stream in response to a determined difference between the value of the parameter and the set-point value of the parameter.

In yet other instances, the method further comprises providing a gaseous component from outside the system or an inert gas.

In some aspects, a method for separating a gas mixture is provided. The method may include providing the gas mixture and feeding the gas mixture to a first membrane separation stage. The first membrane separation stage includes at least one gas separation membrane module configured to separate the gas mixture into a first retentate stream and a first permeate stream. The method further includes providing a sweep stream to a permeate side of the at least one gas separation membrane module of the first membrane separation stage, in which the sweep stream comprises a portion of the first retentate stream.

In some cases, the method includes feeding the first permeate stream to a second membrane separation stage. The second membrane separation stage includes at least one gas separation membrane module configured to separate the first permeate stream into a second retentate stream and a second permeate stream, in which the at least one gas separation membrane module of the second membrane separation stage comprises a membrane having a permeate side and a retentate side. In some such cases, the method also includes providing a controller in electrical communication with one or more measuring devices configured to measure at least one of a parameter of the sweep stream, the first retentate stream, the second retentate stream, the first permeate stream, and the second permeate stream. Such cases may also include determining a value of the at least one parameter, comparing the value of the at least one parameter to a set-point value via the controller, and adjusting a valve in fluid communication with the sweep stream if the at least one parameter is above or below a predetermined threshold value.

In certain cases, the above-described methods may be utilized in any of the systems for separating a gas mixture described herein, including the above-described systems.

In some instances, the sweep stream comprises no more than about 15% of the first retentate stream and the sweep stream is at least about 80% methane.

In any of the above-described aspects and instances, the sweep stream may comprise 0.1% to 100%, or 0.1% to 90%, or 0.1% to 80%, or 0.1% to 70%, or 0.1% to 60%, or 0.1% to 50%, or 0.1% to 40%, or 0.1% to 30%, 0.1% to 25%, or 0.1% to 20%, or 0.1% to 15%, or 0.1% to 10%, or 0.1% to 9% by weight of the retentate stream.

In any of the above-described aspects and instances, the sweep stream may comprise 0.5% to 100%, or 0.5% to 90%, or 0.5% to 80%, or 0.5% to 70%, or 0.5% to 60%, or 0.5% to 50%, or 0.5% to 40%, or 0.5% to 30%, 0.5% to 25%, or 0.5% to 20%, or 0.5% to 15%, or 0.5% to 10%, or 0.5% to 9% by weight of the retentate stream.

In any of the above-described aspects and instances, the sweep stream may comprise 1% to 100%, or 1% to 90%, or 1% to 80%, or 1% to 70%, or 1% to 60%, or 1% to 50%, or 1% to 40%, or 1% to 30%, 1% to 25%, or 1% to 20%, or 1% to 15%, or 1% to 10%, or 1% to 9% by weight of the retentate stream.

In any of the above-described aspects and instances, the sweep stream may comprise 2% to 100%, or 2% to 90%, or 2% to 80%, or 2% to 70%, or 2% to 60%, or 2% to 50%, or 2% to 40%, or 2% to 35%, or 2% to 30%, or 2% to 25%, or 2% to 20%, or 2% to 15%, or 2% to 13%, or 2% to 10%, or 2% to 9% by weight of the retentate stream.

In any of the above-described aspects and instances, the sweep stream may comprise 0.1% to 100%, or 0.1% to 90%, or 0.1% to 80%, or 0.1% to 70%, or 0.1% to 60%, or 0.1% to 50%, or 0.1% to 40%, or 0.1% to 30%, 0.1% to 25%, or 0.1% to 20%, or 0.1% to 15%, or 0.1% to 10%, or 0.1% to 9% by normalized or normal volume of the retentate stream.

In any of the above-described aspects and instances, the sweep stream may comprise 0.5% to 100%, or 0.5% to 90%, or 0.5% to 80%, or 0.5% to 70%, or 0.5% to 60%, or 0.5% to 50%, or 0.5% to 40%, or 0.5% to 30%, 0.5% to 25%, or 0.5% to 20%, or 0.5% to 15%, or 0.5% to 10%, or 0.5% to 9% by normalized or normal volume of the retentate stream.

In any of the above-described aspects and instances, the sweep stream may comprise 1% to 100%, or 1% to 90%, or 1% to 80%, or 1% to 70%, or 1% to 60%, or 1% to 50%, or 1% to 40%, or 1% to 30%, 1% to 25%, or 1% to 20%, or 1% to 15%, or 1% to 10%, or 1% to 9% by normalized or normal volume of the retentate stream.

In any of the above-described aspects and instances, the sweep stream may comprise 2% to 100%, or 2% to 90%, or 2% to 80%, or 2% to 70%, or 2% to 60%, or 2% to 50%, or 2% to 40%, or 2% to 35%, or 2% to 30%, or 2% to 25%, or 2% to 20%, or 2% to 15%, or 2% to 13%, or 2% to 10%, or 2% to 9% by normalized or normal volume of the retentate stream.

In any of the above-described aspects and instances, the amount (e.g., percentage) sweep stream may be defined with reference to a normalized or normal volume of a retentate stream, with reference to a weight of a retentate stream, with reference to a normalized or normal volume of a feed gas stream, and/or with reference to a weight of a feed gas stream.

### BRIEF DESCRIPTION OF THE DRAWINGS

FIG. 1 is a block diagram of a system for separating a gas mixture used in conjunction with a process control system, the system for separating the gas mixture constructed in accordance with the teachings provided herein;
FIG. 2 is a block diagram of another system for separating a gas mixture used in conjunction with a process control system;
FIG. 3 is a block diagram of another system for separating a gas mixture used in conjunction with a process control system;
FIG. 4A is a schematic diagram of a membrane separation stage utilized in the systems for separating a gas mixture of FIGS. 1-3;
FIG. 4B is a schematic diagram of an example process flow in the membrane separation stage of FIG. 4A with various element numbers and lead lines omitted for clarity;
FIG. 5 is a bar graph illustrating CO₂/CH₄ selectivity as a function of the inclusion of a sweep stream of varying composition in a system provided in accordance with the teachings herein;
FIG. 6 is a line graph illustrating the relationship between CO₂ concentration in a retentate and the inclusion of a sweep stream, across a range of feed flow rates and at 6 barG and 8 barG of feed pressure, in a system for separating a gas mixture constructed in accordance with the present disclosure; and
FIG. 7 is a line graph illustrating the relationship between CO₂ concentration in a retentate and the inclusion of a sweep stream of varying composition, across a range of feed flow rates and at 8 barG of feed pressure, in a system for separating a gas mixture constructed in accordance with the present disclosure.

### DETAILED DESCRIPTION

Before any instances of the disclosure are explained in detail, it is to be understood that the disclosure is not limited in its application to the details of construction and the arrangement of components set forth in the following description or illustrated in the following drawings. The disclosure is capable of other instances and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The numerical ranges disclosed herein include all values from, and including, the lower and upper values. For ranges containing explicit values (e.g., a range from 1 to 7, or 2 to 7, or 3 to 5, or 6 to 7), any subrange between any two explicit values is included (e.g., the range 1-7 above includes subranges 1 to 2; 2 to 6; 5 to 7; 3 to 7; 5 to 6; etc.).

It is understood that the sum of the components in each of the mixtures, compositions, streams, lines, and products disclosed herein yields 100 mole percent or about 100 mole percent.

It is understood that the relative amount of gas streams compared to each other encompasses any percent composition used in the art, including, by way of non-limiting example, percent by weight (% w₁/w₂), percent by mass (% m₁/m₂), percent by volume (% normalized v₁/v₂), percent by moles (% n₁/n₂), and percent by concentration (e.g., % M₁/M₂, % m₁/m₂). It is further understood that the subscripts "1" and "2" can refer to any gas stream or component within the system.

The term "line" refers to a physical connection between two points. Nonlimiting examples of suitable lines include fluid conduits, tubes, and pipes. The term "stream" refers to a fluid composition, mixture, or component that may be contained within a line. For example, a feed line may contain a feed stream, where the feed stream comprises a gaseous mixture. It is understood that the terms "line" and "stream" may be used interchangeably herein, such that, for example, the feed line may be referred to herein as the feed stream (and vice versa).

The terms "permeate side" and "retentate side" are used with reference to the gas separation membrane module and refer to whether the gas mixture has passed through the gas separation membrane module. The terms permeate side and retentate side are not necessarily relative to a physical location within the membrane separation stage.

The following discussion is presented to enable a person skilled in the art to make and use instances of the disclosure. Various modifications to the illustrated instances will be readily apparent to those skilled in the art, and the generic principles herein can be applied to other embodiments and applications without departing from embodiments of the disclosure. Thus, instances of the disclosure are not intended to be limited to instances shown but are to be accorded the widest scope consistent with the principles and features disclosed herein. The following detailed description is to be read with reference to the figures, in which like elements in different figures have like reference numerals. The figures, which are not necessarily to scale, depict selected instances and are not intended to limit the scope of instances of the disclosure. Skilled artisans will recognize the examples provided herein have many useful alternatives and fall within the scope of instances of the disclosure.

Additionally, while the following discussion may describe features associated with specific devices or instances, it is understood that additional devices and/or features can be used with the described systems and methods, and that the discussed devices and features are used to provide examples of possible instances, without being limited.

### Systems for Separating a Gas Mixture

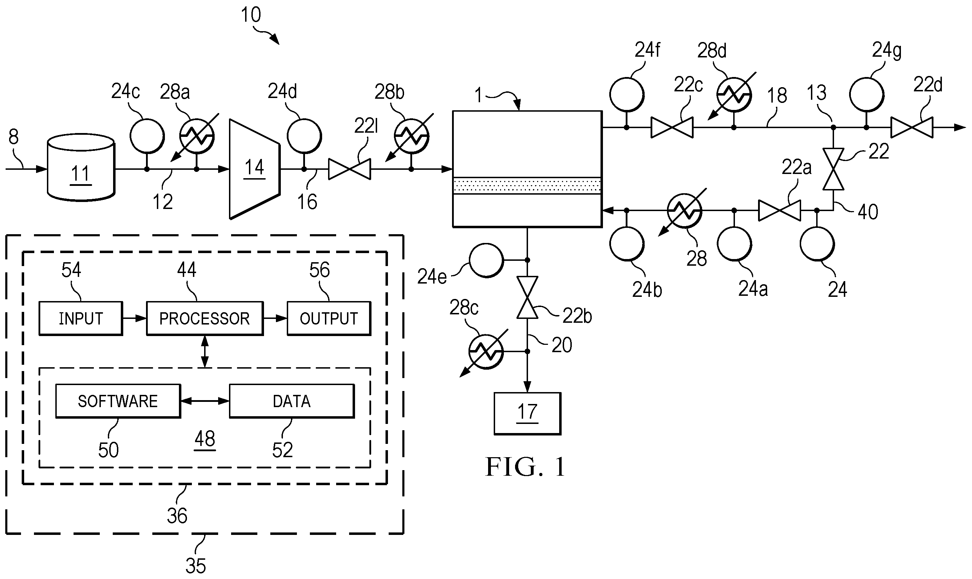

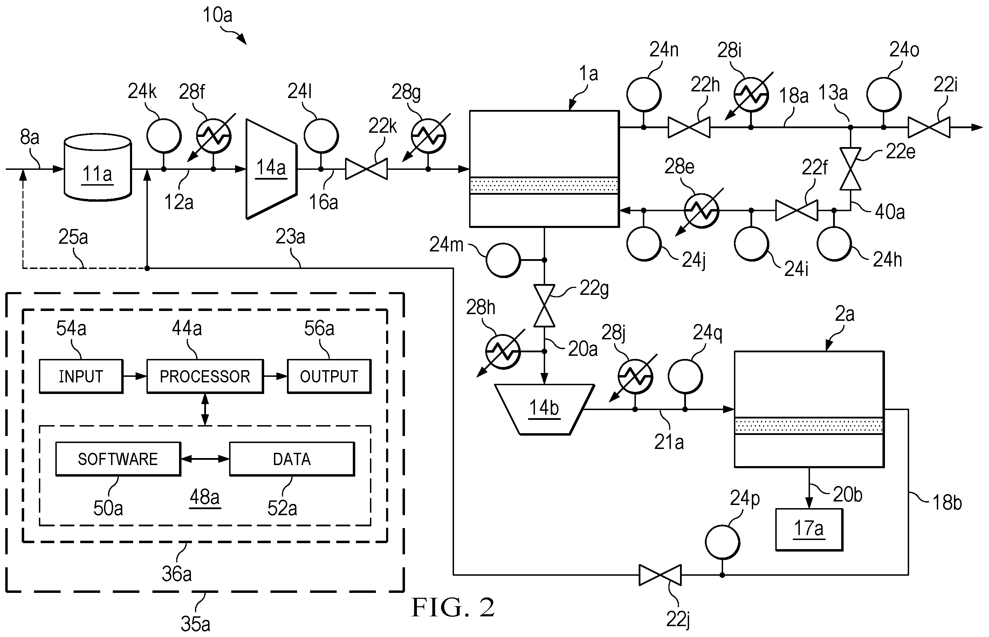

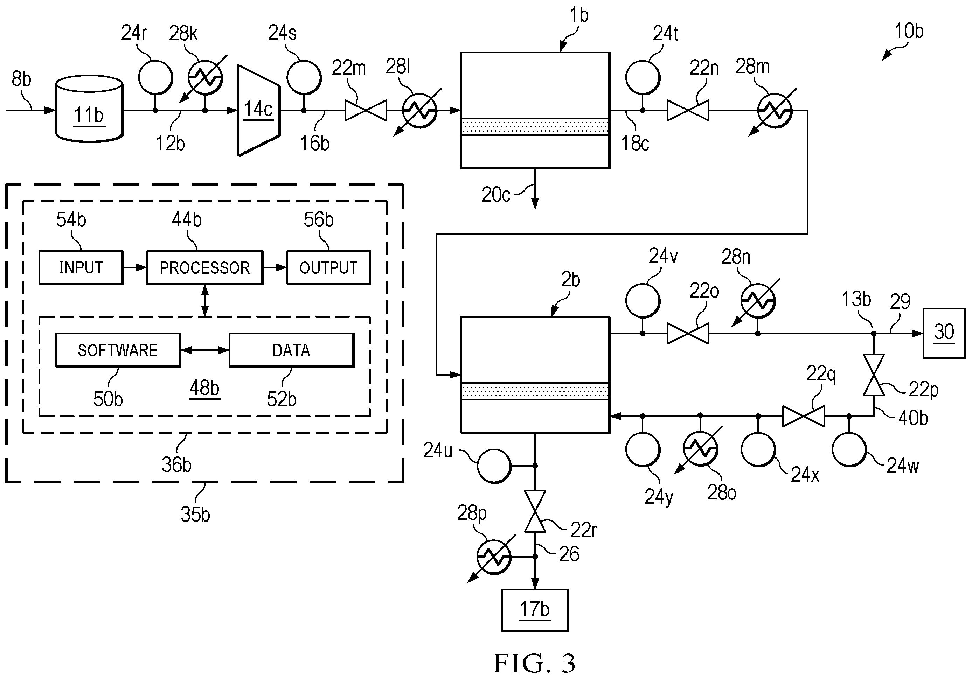

Referring now to FIG. 1, a system 10 for separating a gas mixture is shown. In some instances, the gas mixture may comprise natural gas or biogas. The system 10 may include at least one membrane separation stage (e.g., a membrane separation stage 1) that separates the gas mixture to generate one or more product gas streams. Optionally, the system 10 may include more than one membrane separation stage (e.g., see FIG. 2 and FIG. 3). The system 10 may also include a membrane separation stage 1, an inlet gas line 8, a gas source 11, a feed gas line 12, a gas transport device 14, an adjusted feed stream 16, a retentate stream 18, a permeate stream 20, and a process control system 35 including one or more measuring devices, one or more control devices, and a controller 36.

The membrane separation stage 1 may be in fluid communication with the gas source 11 comprising a natural or man-made reservoir of the gas mixture. In some instances, the gas source 11 may include natural gas or biogas comprising a variety of components, including methane, carbon dioxide, and water. For example, the gas source 11 may comprise, consist essentially of, or consist of methane, carbon dioxide, and water. As an additional example, the gas source 11 may primarily be composed of methane and carbon dioxide. The biogas may be produced from sources such as agricultural waste, manure, municipal waste, plant material, sewage, green waste, food waste, anaerobic digester, or combinations thereof, and transferred to the system 10 for separation. The natural gas may be harvested from naturally occurring wells and provided to the system 10 for separation. In certain instances, the system 10 may comprise a pretreatment unit positioned between the gas source and the membrane separation stage 1, the pretreatment unit designed to remove one or more contaminants from the gas mixture.

The product gas streams generated by the system 10 may at least partially depend on the components of the gas mixture provided to the system 10. For example, if the gas source 11 provides biogas to the membrane separation stage 1, then the system may generate a first product gas stream comprising primarily methane and/or a second product gas stream comprising primarily carbon dioxide. In some instances, the first product gas stream may comprise the retentate stream 18 and the second product gas stream may comprise the permeate stream 20. The product gas streams generated by the system 10 may be collected, stored, vented, further processed, provided to a transport pipeline, provided to an end user, and/or otherwise utilized by the operator of the system 10, as desired.

In some instances, the system 10 includes the gas transport device 14 having a suction side connected to the feed gas line 12 and a discharge side connected to the gas transport adjusted feed stream 16. The gas transport adjusted feed stream 16 places the gas transport device 14 in fluid communication with the membrane separation stage 1, and the feed gas line 12 places the gas transport device 14 in fluid communication with the gas source 11. In general, the gas transport device 14 may be provided as a mechanical device that adjusts (e.g., increases) the pressure of the gas mixture and may comprise a compressor, a blower or the like. In some instances, the gas transport device 14 is configured to feed the gas mixture to the membrane separation stage 1 at a pressure that ranges between 1.5 bar (150 kPa) to 100 bar (10,000 kPa), or between about 1.5 bar (150 kPa) to about 100 bar (10,000 kPa), or more. For example, the gas mixture fed to the membrane separation stage 1 may be imparted with a pressure of 5 bar (500 kPa) to 40 bar (4000 kPa), or 5 bar (500 kPa) to 35 bar (3500 kPa), or 5 bar (500 kPa) to 25 bar (2500 kPa). In other instances, the gas mixture fed to the membrane separation stage 1 may be imparted with a pressure of 1.5 bar (150 kPa) to 100 bar (10,000 kPa), or 5 bar (500 kPa) to 40 bar (4000 kPa), or 5 bar (500 kPa) to 35 bar (3500 kPa), or 5 bar (500 kPa) to 25 bar (2500 kPa). As an additional example, the gas mixture fed to the membrane separation stage 1 may be imparted with a pressure of between 5 bar (500 kPa) to 40 bar (4000 kPa), or 5 bar (500 kPa) to 35 bar (3500 kPa), or 5 bar (500 kPa) to 25 bar (2500 kPa). In other instances, the gas mixture fed to the membrane separation stage 1 may be imparted with a pressure of about 1.5 bar (150 kPa) to about 100 bar (10,000 kPa), or about 5 bar (500 kPa) to about 40 bar (4000 kPa), or about 5 bar (500 kPa) to about 35 bar (3500 kPa), or about 5 bar (500 kPa) to about 25 bar (2500 kPa). In yet other instances, the gas mixture fed to the membrane separation stage 1 may be imparted with a pressure of at least 1.5 bar (150 kPa) or at least about 1.5 bar (150 kPa). It is to be understood that the gas transport device 14 may be configured to feed the gas mixture to the membrane separation stage 1 at a pressure that is somewhat less or greater than the values recited herein. In addition, the gas transport device 14 may be configured to feed the gas mixture to the membrane separation stage 1 at a pressure that falls within a range bounded by any minimum and maximum value as described above. Furthermore, the gas transport device 14 may be configured to feed the gas mixture to the membrane separation stage 1 at a pressure that has a value falling within any of the ranges described herein.

In some instances, the gas transport device 14 may be provided elsewhere in the system 10. For example, the gas transport device 14 may be fluidly coupled to the inlet gas line 8. In other instances, the gas transport device 14 may be omitted from the system 10. In some such instances, the gas source 11 may retain or store the gas mixture at an elevated pressure such that a gas above ambient pressure is provided to the membrane separation stage 1.

Referring again to FIG. 1, the gas separation membrane modules of the membrane separation stage 1 are designed to separate the gas mixture into the retentate stream 18 and the permeate stream 20. The retentate stream 18 includes gas components that do not pass through the membrane(s) of the gas separation membrane modules, and the permeate stream 20 includes gas components that pass through the membrane(s) of the gas separation membrane modules. In instances in which only a single membrane separation stage is provided (e.g., the membrane separation stage 1 of FIG. 1), the retentate stream 18 and/or the permeate stream 20 may provide the product gas(es) generated by the system 10. In certain instances, at least a portion of, substantially all, or all of the retentate stream 18 and/or the permeate stream 20 may be collected, stored, vented, recycled to the system 10, and/or further processed.

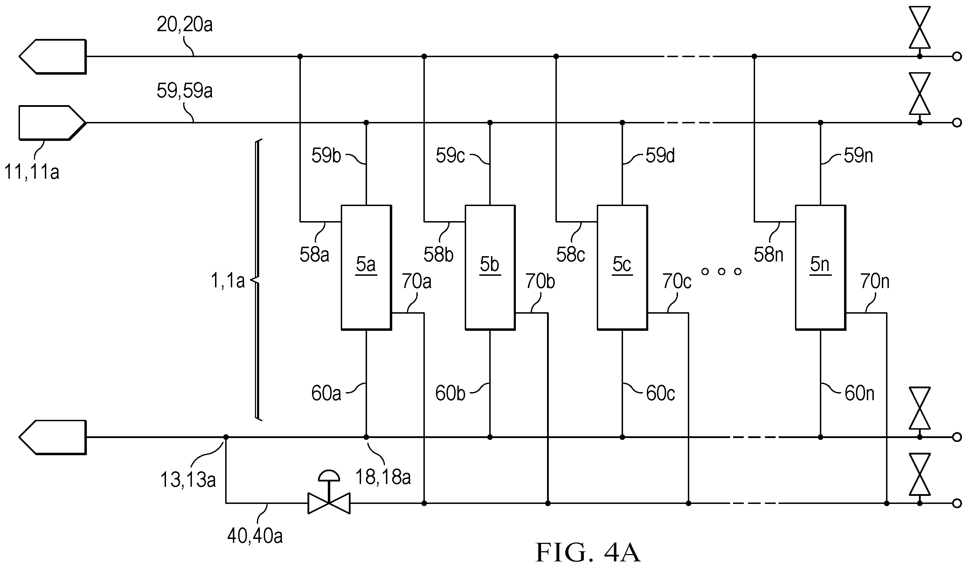

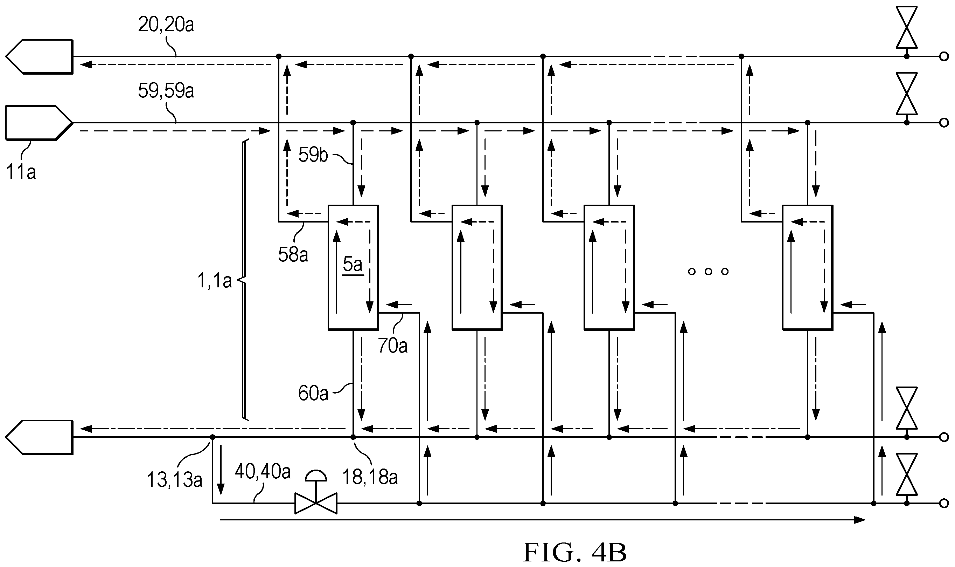

The membrane separation stage 1 may include one or more gas separation membrane modules (see, e.g., FIGS. 4A and 4B) comprising one or more membranes. Generally, the membrane separation stage 1 may utilize membranes designed to separate components of the gas mixture based on solution diffusion across the membrane and/or size exclusion. The membrane itself is typically provided in the form of a thin film that is manufactured from polymers, metals, or ceramics. In some instances, the one or more gas separation membrane modules comprise polymeric membranes, ceramic membranes, metal membranes, or a combination thereof. For example, the one or more gas separation membrane modules may comprise mixed-matrix membranes formed by incorporating an inorganic filler (e.g., a zeolite) into a continuous polymeric matrix (e.g., a polyimide). In other instances, the one or more gas separation membrane modules comprise polymeric membranes. For example, the one or more gas separation membranes used to construct the membrane modules may comprise polyimides, polycarbonates, polyphenyl oxide, polysulfones, cellulose derivatives, polyethylene oxide, and/or combinations thereof. In other instances, the one or more gas separation membrane modules comprise inorganic membranes. For example, the one or more gas separation membrane modules may comprise zeolite membranes. The membrane material may be chosen based on the selectivity of the material for one or more gases in the gas mixture. As an additional example, the one or more gas separation membrane modules may comprise polyimide hollow fiber membranes.

Without being bound to a particular theory, the separation of gases through membranes such as polymeric membranes may be at least partially explained by the solution-diffusion model. The solution-diffusion model involves the (1) dissolution of the gas at the membrane surface on the feed (high-pressure) side of the membrane, (2) diffusion of the gas through the polymer, and (3) desorption of the gas at the permeate (low-pressure) side of the membrane. Generally, steps 1 and 3 are fast relative to step 2, thereby resulting in the diffusion through the membrane being the rate-limiting step in mass transport across a membrane. Differences in the solution and diffusion coefficients for different gas species enable the separation of different gases. The permeability (P) of a particular gas component (x) may be expressed as the product of its solubility coefficient and its diffusion coefficient: Pₓ = SₓDₓ, where Sₓ is the solubility coefficient of the gas component and Dₓ is the diffusion coefficient of the gas component. The permeability (P) of a gas component (x) as well as the thickness of the membrane (lₘ) are both considered to determine the permeance (Q) of the gas component (x) through the membrane and may be expressed as Qₓ = Pₓ/lₘ. The flux (J) of a gas component (x) is the quantity of the gas component (x) that passes through the polymer membrane during one unit of time and area and may be expressed as Jₓ = Qₓ(p_{xfeed} - pₓₚₑᵣₘ), where (p_{xfeed} - pₓₚₑᵣₘ) is the partial pressure difference of the gas component (x) from the feed side to the permeate side of the membrane. Thus, a change in the flux of a gas component may be driven by a change in the partial pressure difference of the gas component across the membrane.

In addition to flux, the solubility of a gas component in a polymer membrane may be affected by properties of the gas, such as the condensability of the gas, and/or properties of the polymer, such as the glass transition temperature (T_{g}) of the polymer, at process conditions. For example, polymers that have glass transition temperatures above a process temperature(s) are glassy at process temperature(s). Glassy polymers or polymers in a glassy state are characterized by poor chain packing and voids between polymer chains, within which gases may adsorb. In a gas separation membrane made from such polymers, at process temperatures, the solubility of gases within the polymer membrane will increase. For example, membranes made from polyimide polymers, such as 3,3'-4,4'- benzophenone tetracarboxylic-dianhydride diaminophenylindane (BTDA-DAPI) (also known as Matrimid^{®} 5218) polymers, are used for gas separation, due in part to the high glass transition temperatures of these polymers.

Furthermore, according to the dual-mode sorption model, which describes gas sorption in a glassy polymer, there are two different groupings of adsorbed gas, at equilibrium with one another, in the glassy polymer: a first grouping of gas that adsorbs within the voids of the glassy polymer and follows a Langmuir curve and a second grouping of gas that is adsorbed into the dense portion of the polymer and follows Henry's law. The dual-mode sorption model may be expressed as C = kp + C'ₕbp/(1+bp), where C is the total concentration of adsorbed gas in the glassy polymer, C'ₕ is the maximum adsorption capacity within the voids, b is the Langmuir affinity constant (ratio of the rate coefficients of adsorption and desorption of gases in the voids), p is pressure, and k is Henry's law sorption coefficient. According to the dual-mode sorption model, at low pressures, the voids within the glassy polymer are filled quickly. As the pressure of the gas increases, the voids become saturated with gas and any additional adsorbed gas is adsorbed into the dense portion of the polymer, following Henry's Law. It is believed that the selectivity of gas separation membranes made of polymers that follow the dual gas sorption model and exhibit dual sorption model behavior may be controlled by operating at lower pressures associated with Langmuir adsorption (in the voids) versus operating at higher pressures associated with Henry's law adsorption.

Taken together, the above illustrates that (1) permeance (i.e., flux) of a species can be increased by increasing the partial pressure difference of species between the feed and permeate side of the membrane and (2) as has been surprisingly found in the context of applying a sweep gas to a membrane for polymers that show dual sorption mode behavior, selectivity can be tuned by operating in the pressure regime where nonlinearity in solubility is present versus the regime where the behavior is mostly linear (i.e. low versus high partial pressures regimes). Thus, it has been discovered that for biogas and natural gas separations, when the permeate side of the membrane is swept with a CH₄-rich, and/or CO₂-lean gas stream, the CO₂ concentration at the membrane surface on the permeate side is forced to go into such a low partial pressure regime such that its solubility becomes high due to the dual-sorption mode. This enhanced solubility at this permeate membrane interface results in competitive sorption that is strongly favored towards CO₂, resulting in increased CO₂ permeability and consequently selectivity.

A measure of the ability of a membrane to separate two gases is the selectivity, α, defined as the ratio of the gas permeabilities, P₁/P₂. Selectivity can also be expressed in terms of the diffusion coefficient of the gas in the membrane D [cm²/s], which is a measure of the gas mobility, and k is Henry's law sorption coefficient, which links the concentration of the gas in the membrane material to the pressure in the adjacent gas [(cm³ (STP))/(cm³ ·cmHg)]. The ratio of the diffusion coefficients of the two gases, D₁/D₂, can be viewed as the mobility selectivity, reflecting the different sizes of the two molecules. The ratio of Henry's law sorption coefficients of the two gases, k₁/k₂, can be viewed as the solubility selectivity, reflecting the relative condensability of the two gases. Depending on the nature of the polymer, either the diffusion or the sorption component of the permeability may dominate.

In some instances, the gas separation membrane of the membrane separation stage 1 is imparted with a CO₂/CH₄ selectivity that is greater than 1. In some instances, the gas separation membrane of the membrane separation stage 1 is imparted with a CO₂/CH₄ selectivity between 1 to 200. In some non-limiting examples, the gas separation membrane of the membrane separation stage 1 is imparted with a CO₂/CH₄ selectivity that ranges between 3 to 85, or 10 to 80, or 20 to 70, or 30 to 60, or 45 to 55. In other non-limiting examples, the gas separation membrane of the membrane separation stage 1 is imparted with a CO₂/CH₄ selectivity between about 1 to about 200, or about 3 to about 85, or about 10 to about 80, or about 20 to about 70, or about 30 to about 60, or about 45 to about 55.

In some instances, the gas separation membrane of the membrane separation stage 1 is imparted with a CO₂/CH₄ selectivity of at least 5, or at least 10, or at least 15, or at least 20, or at least 25, or at least 30, or at least 35, or at least 40, or at least 45, or at least 50, or at least 55, or at least 60, or at least 65, or at least 70, or at least 75, or at least 80, or at least 85, or at least 90, or at least 95, or at least 100, or at least 110, or at least 120, or at least 130, or at least 140, or at least 150, or at least 160, or at least 170, or at least 180, or at least 190, or at least 200. In some instances, the gas separation membrane of the membrane separation stage 1 is imparted with a CO₂/CH₄ selectivity of at least about 5, or at least about 10, or at least about 15, or at least about 20, or at least about 25, or at least about 30, or at least about 35, or at least about 40, or at least about 45, or at least about 50, or at least about 55, or at least about 60, or at least about 65, or at least about 70, or at least about 75, or at least about 80, or at least about 85, or at least about 90, or at least about 95, or at least about 100, or at least about 110, or at least about 120, or at least about 130, or at least about 140, or at least about 150, or at least about 160, or at least about 170, or at least about 180, or at least about 190, or at least about 200. It is to be understood that the gas separation membrane of the membrane separation stage 1 may be imparted with a CO₂/CH₄ selectivity that is somewhat less or greater than the values recited herein. In addition, the gas separation membrane of the membrane separation stage 1 may be imparted with a CO₂/CH₄ selectivity that falls within a range bounded by any minimum and maximum value as described above. Furthermore, the gas separation membrane of the membrane separation stage 1 may be imparted with a CO₂/CH₄ selectivity that falls within any of the ranges described herein.

Referring again to FIG. 1, generally, gas permeation is driven by a partial pressure difference of the gas components from the feed side to the permeate side of the membrane. Several different methods for generating a partial pressure difference across a membrane can be utilized. For example, the pressure of the gas stream fed into a gas separation membrane 1 may be increased. As an additional example, the gas transport device 14 may increase the pressure of the gas mixture in the inlet gas line 8, discharging compressed gas to the gas transport adjusted feed stream 16. As yet another example, the pressure of the gas stream on the permeate side of the membrane may be reduced, such as by using a vacuum or vacuum pump (not illustrated) positioned in the permeate stream 20.

Other methods of a partial pressure difference of the gas components from the feed side to the permeate side of the membrane may also be used. For instance, it has been determined that providing a gas stream (e.g., a retentate stream) as a sweep gas may help generate a larger partial pressure difference of the permeating gas or gases across the membrane. Advantageously, providing a gas stream that is a component of the gas separation system (e.g., at least a portion of a feed stream or a retentate stream), a gas stream sourced from outside of the gas separation system (e.g., at least a portion of a gas sourced from an apparatus, unit, or system that is downstream of the membrane separation apparatus), or an inert gas (e.g. nitrogen gas) sourced from outside the system, as a sweep gas to the permeate side of the membrane separation stage 1 may alter the partial pressure of the gases on the permeate side of the membrane, thereby helping generate a larger partial pressure difference of permeating gas or gases across the membrane. For example, a portion of the retentate stream 18 generated by the membrane separation stage 1 may be provided to the permeate side of the membrane separation stage 1 via a sweep stream 40. Doing so may allow for additional raw biogas to be processed without increasing the number of gas transport devices (e.g., compressors and/or vacuum pumps) provided in the system 10, improve the purity of one or more product gases generated by the system 10, and/or improve recovery of one or more product gases generated by the system 10. In addition, providing a portion of the retentate stream 18 as a sweep gas to the membrane separation stage 1 may also help the system 10 achieve target product gas recovery and purity values without additional membrane separation stages and/or gas transport devices. In some cases, the number of membrane separation stages and/or the number of gas transport devices provided with the system 10 may be reduced and target product gas purity and/or recovery values may still be achieved as compared to systems that do not utilize a sweep gas.

Referring back to FIG. 1, the retentate stream 18 is provided from the retentate side of the membrane separation stage 1. The sweep stream 40 may be in fluid communication with the retentate stream 18 and the permeate side of the membrane separation stage 1. As such, at least a portion of the retentate stream 18 generated by the membrane separation stage 1 may be provided to the permeate side of the membrane separation stage 1. In some instances, such as when the gas source 11 primarily comprises biogas or natural gas and the membrane separation stage 1 includes a polymeric membrane, an inorganic membrane, or a membrane that is a combination thereof, the sweep stream 40 provided to the permeate side of the membrane separation stage 1 is primarily CH₄. Without being bound to a particular theory, it is believed that when the permeate side of the gas separation membrane is swept with a gas stream that is CO₂-lean and/or comprises mostly CH₄, the solubility of CO₂ in the permeate stream 20 increases due to the reduction in the partial pressure of CO₂ in the permeate stream 20 at the membrane surface on the permeate side of the membrane separation stage 1. The increased sorption and/or uptake of CO₂ at this permeate membrane interface results in competitive sorption that strongly favors CO₂, resulting in increased CO₂ permeability and, thereby, increased CO₂ selectivity.

Only a portion of the retentate stream 18 may be provided to the permeate side of the membrane separation stage 1 as a sweep gas. In certain instances, 0.1% to 15% of the volume of the retentate stream 18 [with volume units of, e.g., Nm³/h] may be provided to the permeate side of the membrane separation stage 1 as a sweep gas, although the portion of the retentate stream 18 utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8%, or 8% to 9%, or 9% to 10%, or 10% to 11%, or 11% to 12%, or 12% to 13%, or 13% to 14%, or 14% to 15% of the retentate stream 18 may be utilized as a sweep gas. As an additional example, no more than 15%, or no more than 14%, or no more than 13%, or no more than 12%, or no more than 11%, or no more than 10%, or no more than 9%, or no more than 8%, or no more than 7%, or no more than 6%, or no more than 5%, or no more than 4%, or no more than 3%, or no more than 2%, or no more than 1%, or no more than 0.5%, or no less than 0.1% of the retentate stream 18 may be utilized as a sweep gas. In each instance, the amount of the retentate stream 18 utilized as a sweep gas may be defined in terms of the mass, volume, moles, flow volume, and/or other similar measurements of the retentate stream 18. The remaining portion of the retentate stream 18 not used as a sweep gas may be collected, stored, vented, recycled to the system 10, and/or further processed.

In other instances, about 0.1% to about 15% of the retentate stream 18 may be provided to the permeate side of the membrane separation stage 1 as a sweep gas, although the portion of the retentate stream 18 utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the retentate stream 18 may be utilized as a sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 6%, or no more than about 5%, or no more than about 4%, or no more than about 3%, or no more than about 2%, or no more than about 1%, or no more than about 0.5%, or no less than about 0.1% of the retentate stream 18 may be utilized as a sweep gas. The remaining portion of the retentate stream 18 may be collected, stored, vented, recycled to the system 10, and/or further processed.

In certain cases, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than about 15% of the retentate stream 18 may be utilized as a sweep gas.

In other cases, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the retentate stream 18 may be utilized as a sweep gas.

It is to be understood that the amount of the retentate stream 18 utilized as a sweep gas may fall within a range bounded by any minimum and maximum value as described above. Furthermore, the amount of the retentate stream 18 utilized as a sweep gas may be imparted with a value falling within any of the ranges described herein.

In certain instances, the amount of the sweep gas provided to the permeate side of the membrane separation stage 1 (i.e., the amount of the retentate stream 18 provided to the membrane separation stage 1) can be defined relative to the amount of gas provided to the feed gas line 12. For example, the amount of sweep gas can be represented as a percentage of the overall volume or mass of feed gas supplied to the system 10. It is to be understood that the feed gas supplied to the system 10 may be processed by the membrane separation stage 1 before being utilized as a sweep gas (i.e., that a retentate stream is utilized as the sweep gas). In some such instances, 0.1% to 8% of the feed gas provided to the feed gas line 12 may be utilized as a sweep gas, although the amount of the feed gas that is ultimately utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8% of the feed gas provided to the feed gas line 12 may be utilized as a sweep gas. As an additional example, no more than 8%, or no more than 7%, or no more than 6%, or no more than 5%, or no more than 4.5%, or no more than 4%, or no more than 3.5%, or no more than 3%, or no more than 2%, or no more than 2.5%, or no more than 1%, or no more than 0.5%, or no less than 0.1% of the feed gas provided to the feed gas line 12 may be utilized as a sweep gas.

In other such instances, about 0.1% to about 8% of the feed gas provided to the feed gas line 12 may be utilized as a sweep gas, although the amount of the feed gas utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8% of the feed gas provided to the feed gas line 12 may be utilized as a sweep gas. As an additional example, no more than about 8%, or no more than about 7%, or no more than about 6%, or no more than about 5%, or no more than about 4.5%, or no more than about 4%, or no more than about 3.5%, or no more than about 3%, or no more than about 2.5%, or no more than about 2%, or no more than about 1.5%, or no more than about 1%, or no more than about 0.5%, or no less than about 0.1% of the feed gas provided to the feed gas line 12 may be utilized as a sweep gas.

In certain instances, at least about 0.1%, or at least about 0.5%, or at least about 1%, or at least about 1.5%, or at least about 2%, or at least about 2.5%, or at least about 3%, or at least about 3.5%, or at least about 4%, or at least about 4.5%, or at least about 5%, or at least about 6%, or at least about 7%, or no more than 8% of the feed gas provided to the feed gas line 12 may be utilized as a sweep gas.

In other instances, about 0.1%, or about 0.5%, or about 1%, or about 1.5%, or about 2%, or about 2.5%, or about 3%, or about 3.5%, or about 4%, or about 4.5%, or about 5%, or about 6%, or about 7%, or about 8% of the feed gas provided to the feed gas line 12 may be utilized as a sweep gas.

It is to be understood that the amount of the feed gas provided to the feed gas line 12 that is utilized as a sweep gas may fall within a range bounded by any minimum and maximum value as described above. Furthermore, the amount of the feed gas provided to the feed gas line 12 that is utilized as a sweep gas may be imparted with a value falling within any of the ranges described herein.

In some instances, the composition of the sweep stream 40 may be substantially the same as the composition of the retentate stream 18. In certain instances, the mixture of gases comprising the sweep stream 40 may be the same as, or substantially the same as, the mixture of gases comprising the retentate stream 18.

Optionally, the permeate stream 20 is fed or provided to a carbon dioxide recovery system 17. The carbon dioxide recovery system 17 may prepare purified liquid carbon dioxide from a gas mixture containing carbon dioxide. The obtained purified liquid carbon dioxide may be imparted with a higher purity value than the gas mixture containing carbon dioxide provided to the carbon dioxide recovery system 17. The carbon dioxide recovery system 17 may compress and cool the gas mixture and, optionally, liquefy the gas mixture (e.g., by using purified liquid carbon dioxide as a cooling medium). Any remaining gaseous impurities are either discharged from the liquefied carbon dioxide and vaporized and/or recycled back to the system 10. In addition, purified liquid carbon dioxide may also be withdrawn as a product. The discharged non-condensable gases and vapors may be recycled to fluid stream 8, 8a, 12, 12a, 16, or 16a, vented, or further processed. The carbon dioxide recovery system 17 may include at least one compressor (e.g., two compressors), at least one cooler (e.g., two coolers), and purification equipment coupled to the compressor and the cooler via suitable piping. The components of the carbon dioxide recovery system 17 are not illustrated.

FIG. 1 illustrates the system 10 used in conjunction with an example process control system 35 according to some instances of the present disclosure. The process control system 35 may be configured to control the operation of the system 10. For example, the process control system 35 may assist in starting the process, stopping the process, and adjusting process parameters to change the process performance. In some instances, the process control system 35 may be designed to control the concentration of a selected component in the retentate stream 18 and/or the permeate stream 20, alter or control the amount of gas provided to the sweep stream 40, and/or to monitor and adjust system parameters to control energy consumption, change obtained product purity and/or recovery, and/or to reduce operational costs.

In some instances, the process control system 35 may adjust or control a multitude of process parameters to maintain a product gas purity. In some non-limiting examples, the product gas in the retentate stream 18 comprises mostly methane. For example, the product gas purity of the retentate stream 18 may be controlled by the process control system 35 to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% methane. In some instances, the product gas purity of the retentate stream 18 may be controlled by the process control system 35 to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% methane. In other non-limiting examples, the product gas in the permeate stream 20 comprises mostly carbon dioxide. For example, the product gas purity of the permeate stream 20 may be controlled by the process control system 35 to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% carbon dioxide. In some instances, the product gas purity of the retentate stream 18 may be controlled by the process control system 35 to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% carbon dioxide.

It is to be understood that the product gas purity of the retentate stream 18 and the permeate stream 20 may be somewhat less or greater than the values recited herein. In addition, the product gas purity of the retentate stream 18 and the permeate stream 20 may fall within a range bounded by any minimum and maximum value as described above. Furthermore, the product gas purity of the retentate stream 18 and the permeate stream 20 may have a value falling within any of the ranges described herein.

Referring again to FIG. 1, the process control system 35 of the system 10 includes a controller 36, one or more process measuring devices (e.g., 24, 24a-24g), one or more process control devices (e.g., 14, 22, 22a-22d, 22l, 28, 28a-28d), and suitable connections that allow process information acquired by the one or more measuring devices to be transferred to the controller 36 and output information from the controller 36 to be transferred to the one or more process control devices to perform a process control action. Example process control actions may include using the process control devices to alter one or more of a pressure, flow rate, dewpoint, and/or temperature of a process stream based on one or more measured values from the process measuring devices.

Suitable connections may include transmitters that allow process signals, such as electrical signals or gas pressure signals (e.g., air, nitrogen, etc.), to be transmitted between the controller 36 and the process measuring devices (e.g., 24, 24a-24g) and the process control devices (e.g., 14, 22, 22a-22d, 22l, 28, 28a-28d). The electrical signals may be transferred via a wired connection or through a wireless network connection. Other hardware elements may be included in the process control system 35, for example, transducers, analog-to-digital (A/D) converters, and digital-to-analog (D/A) converters that allow process information to be recognizable in computer form, and computer commands accessible to the process. For visual clarity, the connections between the controller 36, the one or more process measuring devices, and the one or more process control devices have been omitted from FIG. 1.

In some instances, the system 10 includes one or more process measuring devices (e.g., 24, 24a-24g). For example, the system 10 may include one or more sweep gas stream process measuring devices 24, 24a-24b provided in the sweep stream 40. Each of the process measuring devices 24, 24a-24g may be configured to measure a process parameter, or multiple process parameters, such as pressure, temperature, flow, composition, viscosity, humidity, moisture, dewpoint, or density.

In some instances, the one or more process measuring devices 24, 24a-24g includes a pressure measuring device. Suitable pressure measuring devices include, but are not limited to, pressure transducers, diaphragm bellows, Bourdon tubes, strain gauges, piezoelectric sensors, ionization gauges, and combinations thereof.

In some instances, the one or more process measuring devices 24, 24a-24g includes a flow measuring device. Suitable flow measuring devices include, but are not limited to, pitot tubes, orifice flow meters, turbine flow meters, electromagnetic field flow meters, neutron bombardment flow meters, ultrasound flow meters, hot-wire anemometry flow meters, and angular momentum flow meters, Coriolis mass flow meters, and combinations thereof.

In some instances, the one or more process measuring devices 24, 24a-24g includes a temperature measuring device. Suitable temperature measuring devices include, but are not limited to, thermocouples, thermistors (e.g., a resistance sensor), oscillating quartz crystal thermometers, radiation pyrometers, and combinations thereof.

In some instances, the one or more process measuring devices 24, 24a-24g includes a density measuring device. Suitable density measuring devices include, but are not limited to, vibrating tube densometers, X-ray densometers, differential pressure densometers, and combinations thereof.

In some instances, the one or more process measuring devices 24, 24a-24g includes a viscosity measuring device. Suitable viscosity measuring devices include, but are not limited to, capillary viscometers, vibrating ball viscometers, and combinations thereof.

In some instances, the one or more process measuring devices 24, 24a-24g includes a humidity measuring device. Suitable humidity measuring devices include hygrometers.

In some instances, the one or more process measuring devices 24, 24a-24g includes a dewpoint measuring device. Suitable dewpoint measuring devices include those available from Vaisala (Finland) and those available from Michell Instruments (e.g., QMA analyzers).

In some instances, the one or more process measuring devices 24, 24a-24g includes a composition measuring device. Suitable composition measuring devices include, but are not limited to, potentiometry sensors, moisture content sensors (hygrometry or psychrometry), gas chromatography, refractive index devices, ultrasound, spectroscopy systems (e.g., UV, visible, IR, Mossbauer, Raman, atomic-emission device, X-ray device, electron, ion, nuclear magnetic resonance systems), polarography devices, conductimetry devices, mass spectrometry systems, differential thermal analysis devices, thermogravimetric analysis systems, and combinations thereof.

Referring still to FIG. 1, the system 10 includes one or more process control devices, such as valves (e.g., 22, 22a-22d, 22l), temperature control devices (e.g., 28, 28a-28d), and gas transport devices (e.g., 14). In some instances, the system 10 includes one or more valves 22, 22a-22d, 22l that are adjustable between a fully open position and a fully closed position and are used to regulate the pressure of the process streams. For example, the system 10 may include a first sweep stream valve 22 and a second sweep stream valve 22a located in the sweep stream 40 where the first sweep stream valve 22 is upstream of a first sweep stream process measuring device 24 and the second sweep stream valve 22a is downstream of the first sweep stream process measuring device 24. Example valves for use in the system 10 include, without limitation, motor-driven valves, pneumatic valves, and manually adjustable valves.

In certain instances, the controller 36 may control or adjust the valves 22, 22a in response to measured values provided to the controller 36 by one or more process measuring devices (e.g., 24, 24a-24g). For example, the controller 36 may adjust the valves 22, 22a such that additional sweep gas is provided to the permeate side of the membrane separation stage 1 when the purity of a product gas (as measured by, e.g., the process measuring devices 24e, 24f, and/or 24g) drops below a first threshold value. As an additional example, the controller 36 may adjust the valves 22, 22a such that less sweep gas is provided to the permeate side of the membrane separation stage 1 when the purity of a product gas (as measured by, e.g., the process measuring devices 24e, 24f, and/or 24g) is above a threshold value (e.g., the first threshold value or a second threshold value). As yet another example, the controller 36 may adjust the valves 22, 22a such that less sweep gas is provided to the permeate side of the membrane separation stage 1 when the obtained recovery of a product gas (as measured by, e.g., the process measuring devices 24e, 24f, and/or 24g) drops below a third threshold value. As would be understood, these examples are non-limiting, and additional determinations may be made by the controller 36 with regard to opening, closing, and adjusting the valves 22, 22a.

The system 10 may include one or more process measuring devices (e.g., 24, 24a-24g). For example, the system 10 may include a feed gas line measuring device 24c located in the feed gas line 12, a measuring device 24d located in the gas transport adjusted feed stream 16, a permeate measuring device 24e located in the permeate stream 20, a first retentate measuring device 24f located in the retentate stream 18 upstream of a sweep junction 13, a second retentate measuring device 24g located in the retentate stream 18 downstream of the sweep junction 13, and one or more sweep stream process measuring devices 24, 24a, 24b located in the sweep stream 40. The sweep junction 13 may be a fitting designed to minimize turbulence and resistance to flow through the system 10. For example, the sweep junction 13 may be a fitting with a larger radius than a typical elbow or bend fitting. In certain instances, the sweep junction 13 may be further designed to prevent backflow. Each of the process measuring devices 24, 24a-24g may be configured to measure a process parameter, or multiple process parameters, such as pressure, temperature, flow, composition, viscosity, humidity, moisture, dewpoint, or density. In some instances, the one or more sweep stream process measuring devices 24, 24a, 24b may include a flow meter, a pressure transmitter, a temperature sensor, or both.

The system 10 may include one or more temperature control devices (e.g., 28, 28a-28d) that may be used to adjust the temperature (e.g., heat or cool) of the process streams. For example, the system 10 may include one or more of a temperature control device 28a located in the feed gas line 12, a temperature control device 28b located in the gas transport adjusted feed stream 16, a temperature control device 28c located in the permeate stream 20, a temperature control device 28d located in the retentate stream 18, and a sweep stream temperature control device 28 located in the sweep stream 40. The temperature control device 28 located in the sweep stream 40 may be used to increase the temperature of the sweep stream 40 and prevent the sweep stream 40 (as well as other streams, lines, and equipment in the system) from undergoing a phase change (e.g., solidifying and/or condensing) from a gas to a non-gas (e.g., liquid or solid) due to the reduction of pressure of the sweep stream 40 as it flows from the retentate stream 18 and to the permeate side of the membrane separation stage 1. Depending on the configuration of the sweep stream 40 and other process design requirements, it may be advantageous to locate the temperature device 28 and other relevant process measuring devices in the sweep stream 40 to locations upstream of valve 22 or valve 22a. Suitable temperature control devices 28, 28a-28d include devices that heat or cool the process stream, such as heat exchangers or electric heaters.

The controller 36 may include a processor 44 and a memory 48 that includes software 50 and data 52 and is designed for storage and retrieval of processed information to be processed by the processor 44. The processor 44 includes an input 54 that is configured to receive process signals from the one or more process measuring devices (24, 24a-24g) and from the one or more process control devices (e.g., 14, 22, 22a-22d, 22l, 28, 28a-28d) via the input 54. The controller 36 may operate autonomously or semi-autonomously, or it may read executable software instructions from the memory 48, 48a or a computer-readable medium (e.g., a hard drive, a CD-ROM, flash memory), or may receive instructions via the input 54 from a user, or another source logically connected to a computer or device, such as another networked computer or server. For example, the server may be used to control the system 10 via the controller 36 on-site or remotely.

The processor 44 may process the process signals to generate an output 56a, which may take the form of a process control action. Example process control actions may include sending signals to the one or more process control devices (e.g., 14, 22, 22a-22d, 22l, 28, 28a-28d) to effectuate a change in one or more process parameters (e.g., pressure, flow rate, and/or temperature) of one or more process streams in the system 10.

In some instances, the controller 36 may include programming to adjust the process parameters in the system 10 using the one or more process control devices (e.g., 14, 22, 22a-22d, 22l, 28, 28a-28d) in response to measured values obtained from the one or more process measuring devices (e.g., 24, 24a-24g) to maintain a selected set-point, such as a desired gas composition (e.g., at least 95% methane) and/or the flow rate of the sweep stream 40 and/or the amount of the retentate stream 18 provided to the sweep stream 40.

For example, the controller 36 may be programmed to monitor one or more process parameters (e.g., dewpoint, flow rate, a concentration of gas components at or near the point where the sweep stream is fed into a membrane stage) in the sweep stream 40 using the one or more sweep stream process measuring devices 24, 24a, 24b located in the sweep stream 40. Once a change in the process parameter is detected or a user-specified threshold has been reached, the pressure of the sweep stream 40 may be adjusted using, for example, one or more of the first sweep stream valve 22 and the second sweep stream valve 22a to maintain the selected set-point. The selected set-point in the sweep stream 40 may thus be maintained by adjusting the pressure in the sweep stream 40 using one or more of the sweep stream valves 22. Alternatively, or additionally, the feed gas line measuring device 24c may be used to monitor one or more process parameters in the feed gas line 12, the measuring device 24d located in the gas transport adjusted feed stream 16 may be used to monitor one or more process parameters in the gas transport adjusted feed stream 16, the permeate measuring device 24e may be used to monitor one or more process parameters in the permeate stream 20, and the first and second retentate measuring devices 24f, 24g located respectively upstream of the sweep junction 13 and downstream of the sweep junction 13 in the retentate stream 18 may be used to monitor one or more process parameters in the retentate stream 18.

In some instances, the controller 36 may be in electrical communication with the one or more sweep stream process measuring devices 24, 24a, 24b and the one or more valves 22, 22a, located in the sweep stream 40 where the controller 36 is configured to maintain or adjust the position of the one or more valves 22, 22a located in the sweep stream 40 in response to the measured parameter.

In some instances, the controller 36 may include programming to adjust the process parameters in the system 10 using the one or more process control devices (e.g., 14, 22, 22a-22d, 22l, 28, 28a-28d) in response to measured values obtained from the one or more process measuring devices (e.g., 24, 24a-24g) to maintain a selected set-point in the retentate stream 18 such as a desired pressure, a desired component composition, and/or a desired flow rate.

For example, the controller 36 may be programmed to monitor one or more process parameters in the retentate stream 18 (e.g., pressure, etc.) using the first retentate measuring device 24f located in the retentate stream 18 upstream of the sweep junction 13. Once a change in the process parameter is detected or a user-specified threshold has been reached, the pressure of the retentate stream 18 upstream of the sweep junction 13 may be adjusted using the first retentate valve 22c to maintain the selected set-point. Alternatively, or additionally, the second retentate measuring device 24g located in the retentate stream 18 downstream of the sweep junction 13 may be used to monitor one or more process parameters in the retentate stream 18. Once a change in the process parameter is detected or a user-specified threshold has been reached, the pressure of the retentate stream 18 downstream of the sweep junction 13 may be adjusted using the second retentate valve 22d to maintain the selected set-point.

In some instances, the controller 36 may include programming to adjust process parameters in the system 10 using the one or more process control devices (e.g., 14, 22, 22a-22d, 22l, 28, 28a-28d) in response to measured values obtained from the one or more process measuring devices (e.g., 24, 24a-24g) to maintain a selected set-point in the gas transport adjusted feed stream 16 such as a desired pressure, a desired component composition, and/or a desired flow rate.

In some instances, the controller 36 may include programming to adjust process parameters in the system 10 using the one or more process control devices (e.g., 14, 22, 22a-22d, 22l, 28, 28a-28d) in response to measured values obtained from the one or more measuring devices (e.g., 24, 24a-24g) to maintain a selected set-point in the permeate stream 20, such as a desired pressure, a desired component composition, and/or a desired flow rate.

For example, the controller 36 may be programmed to acquire one or more process parameters in the permeate stream 20 using the permeate measuring device 24e. Once a change is detected or a user-specified threshold has been reached, the pressure of the permeate stream 20 may be adjusted using, for example, the permeate valve 22b to maintain the selected set-point. In one non-limiting example, the composition (e.g., methane concentration) of the permeate stream 20 is measured and controlled to maintain the selected set-point by adjusting the pressure in the feed gas line 12 using the gas transport device 14.

In some instances, the controller 36 may include programming to adjust process parameters in the system 10 using the one or more process control devices (e.g., 14, 22, 22a-22d, 22l, 28, 28a-28d) in response to measured values obtained from the one or more measuring devices (e.g., 24, 24a-24g) to maintain a selected set-point in the inlet gas line 8 and/or the feed gas line 12 such as a desired pressure, a desired component composition, and/or a desired flow rate.

In some instances, the controller 36 may include programming to adjust process parameters in the system 10 using the one or more process control devices (e.g., 14, 22, 22a-22d, 22l, 28, 28a-28d) in response to measured values obtained from the one or more measuring devices (e.g., 24, 24a-24g) to maintain a selected temperature set-point for the membrane separation stage 1.

For example, the controller 36 may be programmed to acquire one or more process parameters in the system 10 using one or more of the process measuring devices (e.g., 24, 24a-24g) and adjust the operation temperature of the membrane separation stage 1 using the temperature control device 28a located in the feed gas line 12, the temperature control device 28b located in the gas transport adjusted feed stream 16, and/or the temperature control device 28d located in the retentate stream 18 to obtain a selected temperature set-point.

It is to be understood that any of the parameters associated with the various gas streams of the system 10 may be determined, measured, altered, and/or adjusted more than once. For example, a first measurement of the one or more values or parameters of a gas stream may be carried out at a first time period, followed by a second measurement carried out at a second time period, where the amount of time that elapses between the first time period and the second time period is determined by the predetermined time interval or another predetermined operational condition. In each instance, such measurements may be carried out by one or more measuring devices (e.g., the one or more process measuring devices 24, 24a-24g) and then received and stored by a controller (e.g., the controller 36). In addition, a process control system (e.g., the process control system 35) may take a first action to adjust one or more parameters of a gas stream using one or more process control devices (e.g., the one or more process control devices 14, 22, 22a-22d, 22l, 28, 28a-28d) at a third time period, followed by a second action carried out at a fourth time period, where the amount of time that elapses between the third time period and the fourth time period is determined by the predetermined time interval or another predetermined operational condition.

Further, in some instances, one or more of the above processes can use machine learning, (ML), artificial intelligence (AI), or similar techniques, to iteratively train the controller 36 and improve the performance of the system based on one or more feedback parameters, characteristics, or similar. For example, in some instances, ML/AI can be used to predict an optimal pressure for the retentate stream 18 based on system data such as pressure, volume, mass, temperature, user preferences, etc. In some instances, ML/AI can be used to determine or predict amounts of a sweep gas to supply from the retentate stream 18 or another source. Thus, the amount of sweep gas returned to the membrane separation stage 1 in the sweep stream 40 can be optimized. This can be beneficial because the performance and efficiency of the membrane separation stage can be optimized while maintaining or improving one or more outputs of the system 10.

Referring now to FIG. 2, a system for separating a gas mixture, a system 10a, may be provided in the form of two or more membrane separation stages. Generally, when comparing the system 10a of FIG. 2 and the system 10 of FIG. 1, components having similar names and element numbers may have substantially similar structures and functions. For example, like the system 10, the system 10a may include an inlet gas line 8a, a gas source 11a, a first gas transport device 14a, a first gas transport adjusted feed stream 16a, a first membrane separation stage 1a, a first retentate stream 18a, a first permeate stream 20a, a process control system 35a, and a sweep stream 40a. However, unlike the illustrated version of the system 10 in FIG. 1, the system 10a may include a second membrane separation stage 2a in addition to the first membrane separation stage 1a. Moreover, in the system 10a, a sweep stream 40a is provided to the permeate side of the first membrane separation stage 1a. Furthermore, in addition to the components described with reference to the system 10, the system 10a may also include a second gas transport device 14b, a second gas transport adjusted feed stream 21a, a second membrane separation stage 2a, a second permeate stream 20b, and a second retentate stream 18b, additional process measuring devices, and/or additional process control devices.

In some examples, the system 10a may include at least three membrane separation stages (the additional membrane separation stages are not illustrated). If at least three membrane separation stages are provided in the system 10a, the membrane separation stages may be arranged in series, in parallel, or in series and in parallel. In each instance, at least one membrane separation stage (e.g., the first membrane separation stage 1a) may be coupled to a sweep stream such that a portion of the retentate generated by the membrane separation stage may be provided to the permeate side of said membrane separation stage.

Referring again to FIG. 2, in some instances, the first permeate stream 20a may be fed to the second gas transport device 14b. The second gas transport device 14b may compress the CO₂-rich first permeate stream 20a to yield the second gas transport adjusted feed stream 21a, which may then be fed into the second membrane separation stage 2a. Similar to the membrane separation stage 1 in FIG. 1 and the first membrane separation stage 1a in FIG. 2, the second membrane separation stage 2a may include one or more gas separation membrane modules that may be designed to separate the second gas transport adjusted feed stream 21a into a second permeate stream 20b and a second retentate stream 18b. The second permeate stream 20b may be removed as a product, further processed, or discarded. The second retentate stream 18b may be removed as a product, further processed, recycled back to the feed gas line 12a via a recycle line 23a, and/or recycled back to the inlet gas line 8a upstream of the gas source 11a via an optional recycle line 25a. **In** some instances, the pressure and/or flow rate of the recycle stream 23a may be adjusted or controlled via a process control device in fluid communication with the recycle stream 23a (i.e., a recycle stream valve 22j). **In** addition, a process measuring device 24p may be in fluid communication with the recycle stream 23a and may monitor various parameters of the recycle stream 23a.

In some instances, the second permeate stream 20b may be fed or provided to the carbon dioxide recovery system 17a. The carbon dioxide recovery system 17a may prepare purified liquid carbon dioxide from a gas mixture containing carbon dioxide. The obtained purified liquid carbon dioxide may be imparted with a higher purity value than the gas mixture containing carbon dioxide provided to the carbon dioxide recovery system 17a. The carbon dioxide recovery system 17a may compress and cool the gas mixture and, optionally, liquefy the gas mixture (e.g., by using purified liquid carbon dioxide as a cooling medium), whereupon gaseous impurities are either discharged from the liquefied carbon dioxide and vaporized and/or recycled back to the system 10a. In addition, the purified liquid carbon dioxide may be withdrawn as a product. The carbon dioxide recovery system 17a may include at least one compressor (e.g., two compressors), at least one cooler (e.g., two coolers), and purification equipment coupled to the compressor and the cooler via suitable piping (the components of the carbon dioxide recovery system 17a are not illustrated).

In some instances, the controller 36a may adjust or control a multitude of process parameters to maintain a product gas purity. In some non-limiting examples, the product gas in the first retentate stream 18a may comprise mostly methane. For example, the product gas purity of the first retentate stream 18a may be controlled by the process control system 35a to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% methane. In some instances, the product gas purity of the first retentate stream 18a may be controlled by the process control system 35a to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% methane.

In other non-limiting examples, the product gas in the second permeate stream 20b may comprise mostly carbon dioxide. For example, the product gas purity of the second permeate stream 20b may be controlled by the process control system 35a to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% carbon dioxide. In some instances, the product gas purity of the retentate stream 18a, 18b may be controlled by the process control system 35a to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% carbon dioxide.

It is to be understood that the product gas purity of the retentate stream 18a, 18b and the second permeate stream 20b may be somewhat less or greater than the values recited herein. In addition, the product gas purity of the retentate stream 18a, 18b and the second permeate stream 20b may fall within a range bounded by any minimum and maximum value as described above. Furthermore, the product gas purity of the retentate stream 18a, 18b and the second permeate stream 20b may have a value falling within any of the ranges described herein.

Referring still to FIG. 2, in certain instances, about 0.1% to about 15% of the first retentate stream 18a may be provided to the permeate side of the first membrane separation stage 1a as a sweep gas, although the portion of the first retentate stream 18a utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the first retentate stream 18a may be utilized as sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 5%, or no more than about 4%, or no more than about 3%, or no more than about 2%, or no more than about 1%, or no more than about 0.5%, or no less than 0.1% of the first retentate stream 18a may be utilized as a sweep gas. The remaining portion of the first retentate stream 18a may be collected, stored, vented, recycled to the system 10a, and/or further processed.

In certain cases, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than about 15% of the retentate stream 18a may be utilized as a sweep gas.

In other cases, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the retentate stream 18a may be utilized as a sweep gas.

It is to be understood that the amount of the first retentate stream 18a utilized as a sweep gas may fall within a range bounded by any minimum and maximum value as described above. Furthermore, the amount of the first retentate stream 18a utilized as a sweep gas may be imparted with a value falling within any of the ranges described herein.

In certain instances, the amount of the sweep gas provided to the permeate side of the first membrane separation stage 1a (i.e., the amount of the retentate stream 18a provided to the first membrane separation stage 1a) can be defined relative to the amount of gas provided to the feed gas line 12a. It is to be understood that the feed gas supplied to the system 10a may be processed by the first membrane separation stage 1a before being utilized as a sweep gas (i.e., that a retentate stream is utilized as the sweep gas). For example, the amount of sweep gas can be represented as a percentage of the overall volume or mass of feed gas supplied to the system 10a. In some such instances, 0.1% to 8% of the feed gas provided to the feed gas line 12a may be utilized as a sweep gas, although the amount of the feed gas that is ultimately utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8% of the feed gas provided to the feed gas line 12 may be utilized as a sweep gas. As an additional example, no more than 8%, or no more than 7%, or no more than 6%, or no more than 5%, or no more than 4.5%, or no more than 4%, or no more than 3.5%, or no more than 3%, or no more than 2.5%, or no more than 2%, or no more than 1.5%, or no more than 1%, or no more than 0.5%, or no less than 0.1% of the feed gas provided to the feed gas line 12a may be utilized as a sweep gas.

In other such instances, about 0.1% to about 8% of the feed gas provided to the feed gas line 12a may be utilized as a sweep gas, although the amount of the feed gas utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8% of the feed gas provided to the feed gas line 12a may be utilized as a sweep gas.

As an additional example, no more than about 8%, or no more than about 7%, or no more than about 6%, or no more than about 5%, or no more than about 4.5%, or no more than about 4%, or no more than about 3.5%, or no more than about 3%, or no more than about 2.5%, or no more than about 2%, or no more than about 1.5%, or no more than about 1%, or no more than about 0.5%, or no less than about 0.1% of the feed gas provided to the feed gas line 12a may be utilized as a sweep gas.

In certain instances, at least about 0.1%, or at least about 0.5%, or at least about 1%, or at least about 1.5%, or at least about 2%, or at least about 2.5%, or at least about 3%, or at least about 3.5%, or at least about 4%, or at least about 4.5%, or at least about 5%, or at least about 6%, or at least about 7%, or no more than 8% of the feed gas provided to the feed gas line 12a may be utilized as a sweep gas.

In other instances, about 0.1%, or about 0.5%, or about 1%, or about 1.5%, or about 2%, or about 2.5%, or about 3%, or about 3.5%, or about 4%, or about 4.5%, or about 5%, or about 6%, or about 7%, or about 8% of the feed gas provided to the feed gas line 12a may be utilized as a sweep gas.

It is to be understood that the amount of the feed gas provided to the feed gas line 12a that is utilized as a sweep gas may fall within a range bounded by any minimum and maximum value as described above. Furthermore, the amount of the feed gas provided to the feed gas line 12a that is utilized as a sweep gas may be imparted with a value falling within any of the ranges described herein.

In some instances, the composition of the sweep stream 40a may be substantially the same as the composition of the first retentate stream 18a.

In some instances, the process control system 35a may include a controller 36a, one or more process measuring devices (e.g., 24h-24q), one or more process control devices (e.g., 14a, 14b, 22e-22k, 28, 28a-28j), and suitable connections that allow process information acquired by the one or more measuring devices to be transferred to the controller 36a and output information from the controller 36a to be transferred to the one or more process control devices to perform a process control action. Example process control actions may include using the process control devices to alter one or more of a pressure, flow rate, dewpoint, and/or temperature of a process stream based on one or more measured values from the process measuring devices.

Suitable connections may include transmitters that allow process signals, such as electrical signals or gas pressure signals (e.g., air, nitrogen, etc.), to be transmitted between the controller 36a and the process measuring devices (e.g., 24h-24q) and the process control devices (e.g., 14a, 14b, 22e-22k, 28e-28j). The electrical signals may be transferred via a wired connection or through a wireless network connection. Other hardware elements may be included in the process control system 35a, for example, transducers, analog-to-digital (A/D) converters, and digital-to-analog (D/A) converters that allow process information to be recognizable in computer form, and computer commands accessible to the process. For visual clarity, the connections between the controller 36a, the one or more measuring devices, and the one or more process control devices have been omitted from FIG. 2.

In some instances, the system 10a may include one or more process measuring devices (e.g., 24h-24q). For example, the system 10a may include one or more sweep stream process measuring devices 24h-24j provided in the sweep stream 40, 40a. Each of the process measuring devices 24h-24q may be configured to measure a process parameter, or multiple process parameters, such as pressure, temperature, flow, composition, viscosity, humidity, moisture, dewpoint, or density.

In some instances, the one or more process measuring devices 24h-24q may include a pressure measuring device. Suitable pressure measuring devices include, but are not limited to, pressure transducers, diaphragm bellows, Bourdon tubes, strain gauges, piezoelectric sensors, ionization gauges, and combinations thereof.

In some instances, the one or more process measuring devices 24h-24q may include a flow measuring device. Suitable flow measuring devices include, but are not limited to, pitot tubes, orifice flow meters, turbine flow meters, electromagnetic field flow meters, neutron bombardment flow meters, ultrasound flow meters, hot-wire anemometry flow meters, and angular momentum flow meters, Coriolis mass flow meters, and combinations thereof.

In some instances, the one or more process measuring devices 24h-24q may include a temperature measuring device. Suitable temperature measuring devices include, but are not limited to, thermocouples, thermistors (e.g., a resistance sensor), oscillating quartz crystal thermometers, radiation pyrometers, and combinations thereof.

In some instances, the one or more process measuring devices 24h-24q may include a density measuring device. Suitable density measuring devices include, but are not limited to, vibrating tube densometers, X-ray densometers, differential pressure densometers, and combinations thereof.

In some instances, the one or more process measuring devices 24h-24q may include a viscosity measuring device. Suitable viscosity measuring devices include, but are not limited to, capillary viscometers, vibrating ball viscometers, and combinations thereof.

In some instances, the one or more process measuring devices 24h-24q may include a humidity measuring device. Suitable humidity measuring devices include hygrometers.

In some instances, the one or more process measuring devices 24h-24q may include a dewpoint measuring device. Suitable dewpoint measuring devices include those available from Vaisala (Finland) and those available from Michell Instruments (e.g., QMA analyzers).

In some instances, the one or more process measuring devices 24h-24q may include a composition measuring device. Suitable composition measuring devices include, but are not limited to, potentiometry sensors, moisture content sensors (hygrometry or psychrometry), gas chromatography, refractive index devices, ultrasound, spectroscopy systems (e.g., UV, visible, IR, Mossbauer, Raman, atomic-emission device, X-ray device, electron, ion, nuclear magnetic resonance systems), polarography devices, conductimetry devices, mass spectrometry systems, differential thermal analysis devices, thermogravimetric analysis systems, and combinations thereof.

Referring still to FIG. 2, the system 10a may include one or more process control devices, such as valves (e.g., 22e-22k), temperature control devices (e.g., 28e-28j), and/or gas transport devices (e.g., 14a, 14b). In some instances, the system 10a may include one or more valves 22e-22k that are adjustable between a fully open position and a fully closed position and are used to regulate the pressure of the process streams. For example, the system 10a may include a first sweep stream valve 22e and a second sweep stream valve 22f located in the sweep stream 40a where the first sweep stream valve 22e is upstream of a first sweep stream process measuring device 24h and the second sweep stream valve 22f is downstream of the first sweep stream process measuring device 24h. In some instances, only the first sweep stream valve 22e or only the second sweep stream valve 22f may be provided. In other instances, another valve or an additional valve may be positioned in either the first retentate stream 18a or the sweep stream 40a to control the amount of sweep gas provided to the permeate side of the membrane separation stage 1. Example valves for use in the system 10a include, without limitation, motor-driven valves, pneumatic valves, and manually adjustable valves.

The system 10a may include one or more process measuring devices (e.g., 24h-24q). For example, the system 10a may include a feed gas line measuring device 24k located in the feed gas line 12a, a measuring device 24l located in the gas transport adjusted feed stream 16, 16a, a permeate measuring device 24e, 24m located in the first permeate stream 20a, a first retentate measuring device 24n located in the first retentate stream 18a upstream of the sweep junction 13a, a second retentate measuring device 24o located in the first retentate stream 18a downstream of the sweep junction 13a, and one or more sweep stream process measuring devices 24h, 24i, 24j located in the sweep stream 40a. Each of the process measuring devices 24h-24q may be configured to measure a process parameter, or multiple process parameters, such as pressure, temperature, flow, composition, viscosity, humidity, moisture, dewpoint, or density. In some instances, the one or more sweep stream process measuring devices 24h, 24i, 24j may include a flow meter, a temperature sensor, or both.

The system 10a may include one or more temperature control devices (e.g., 28e-28j) that may be used to adjust the temperature (e.g., heat or cool) of the process streams. For example, the system 10a may include one or more of a temperature control device 28f located in the feed gas line 12a, a temperature control device 28g located in the gas transport adjusted feed stream 16a, a temperature control device 28h located in the first permeate stream 20a, a temperature control device 28i located in the first retentate stream 18a, and a sweep stream temperature control device 28e located in the sweep stream 40a. The temperature control device 28e located in the sweep stream 40a may be used to increase the temperature of the sweep stream 40a and prevent the sweep stream 40a, or components within the sweep stream 40a, from undergoing a phase change (e.g., solidifying and/or condensing) from a gas to a non-gas (e.g., liquid or solid) due to the reduction of pressure experienced by the sweep gas in the sweep line 40a. Suitable temperature control devices 28e-28j include devices that heat or cool the process stream, such as heat exchangers or electric heaters.

The controller 36a may include a processor 44a and a memory 48a that includes software 50a and data 52a and is designed for storage and retrieval of processed information to be processed by the processor 44a. The processor 44a includes an input 54a that is configured to receive process signals from the one or more process measuring devices (24h-24q) and from the one or more process control devices (e.g., 14a, 14b, 22e-22k, 28e-28j) via the input 54a. The controller 36a may function substantially similarly to the controller 36. The controller 36a may operate autonomously or semi-autonomously, or it may read executable software instructions from the memory 48a or a computer-readable medium (e.g., a hard drive, a CD-ROM, flash memory), or may receive instructions via the input 54a from a user, or another source logically connected to a computer or device, such as another networked computer or server. For example, the server may be used to control the system 10a via the controller 36a on-site or remotely.

The processor 44a may process the process signals to generate an output 56a, which may take the form of a process control action. Example process control actions may include sending signals to the one or more process control devices (e.g., 14a, 14b, 22e-22k, 28e-28j) to effectuate a change in one or more process parameters (e.g., pressure, flow rate, composition, membrane surface area, and/or temperature) of one or more process streams in the system 10a.

In some instances, the controller 36a may include programming to adjust the process parameters in the system 10a using the one or more process control devices (e.g., 14a, 14b, 22e-22k, 28e-28j) in response to measured values obtained from the one or more process measuring devices (e.g., 24h-24q) to maintain a selected set-point, such as a desired gas composition (e.g., at least 95% methane) and/or the flow rate of the sweep stream 40a and/or the amount of the retentate stream 18 provided to the sweep stream 40a.

For example, the controller 36a may be programmed to monitor one or more process parameters (e.g., dewpoint, flow rate) in the sweep stream 40a using the one or more sweep stream process measuring devices 24h, 24i, 24j located in the sweep stream 40a. Once a change in the process parameter is detected or a user-specified threshold has been reached, the pressure of the sweep stream 40a may be adjusted using one or more of the first sweep stream valve 22e and the second sweep stream valve 22f to maintain the selected set-point. The selected set-point in the sweep stream 40a may thus be maintained by adjusting the pressure in the sweep stream 40a using one or more of the sweep stream valves 22e, 22f. Alternatively, or additionally, the feed gas line measuring device 24k may be used to monitor one or more process parameters in the feed gas line 12a, the measuring device 24l located in the gas transport adjusted feed stream 16a may be used to monitor one or more process parameters in the gas transport adjusted feed stream 16a, the permeate measuring device 24e, 24m may be used to monitor one or more process parameters in the first permeate stream 20a, and the first and second retentate measuring devices 24f, 24g, 24n, 24o located respectively upstream of the sweep junction 13a and downstream of the sweep junction 13a in the first retentate stream 18a may be used to monitor one or more process parameters in the first retentate stream 18a.

In some instances, the controller 36a may be in electrical communication with the one or more sweep stream process measuring devices 24h, 24i, 24j and the one or more valves 22e, 22f located in the sweep stream 40a where the controller 36a may be configured to maintain or adjust the position of the one or more valves 22e and 22f located in the sweep stream 40a in response to the measured parameter.

In some instances, the controller 36a may include programming to adjust the process parameters in the system 10a using the one or more process control devices (e.g., 14a, 14b, 22e-22k, 28e-28j) in response to measured values obtained from the one or more process measuring devices (e.g., 24h-24q) to maintain a selected set-point in the retentate stream 18a such as a desired pressure, a desired component composition, and/or a desired flow rate.

For example, the controller 36a may be programmed to monitor one or more process parameters in the first retentate stream 18a (e.g., pressure, etc.) using the first retentate measuring device 24n located in the first retentate stream 18a upstream of the sweep junction 13a. Once a change in the process parameter is detected or a user-specified threshold has been reached, the pressure of the first retentate stream 18a upstream of the sweep junction 13a may be adjusted using the first retentate valve 22h to maintain the selected set-point. Alternatively, or additionally, the second retentate measuring device 24o located in the first retentate stream 18a downstream of the sweep junction 13a may be used to monitor one or more process parameters in the first retentate stream 18a. Once a change in the process parameter is detected or a user-specified threshold has been reached, the pressure of the first retentate stream 18a downstream of the sweep junction 13a may be adjusted using the second retentate valve 22i to maintain the selected set-point.

In some instances, the controller 36a may include programming to adjust process parameters in the system 10a using the one or more process control devices (e.g., 14a, 14b, 22e-22k, 28e-28j) in response to measured values obtained from the one or more process measuring devices (e.g., 24h-24q) to maintain a selected set-point in the gas transport adjusted feed stream 16a such as a desired pressure, a desired component composition, and/or a desired flow rate.

In some instances, the controller 36a may include programming to adjust process parameters in the system 10a using the one or more process control devices (e.g., 14a, 14b, 22e-22k, 28e-28j) in response to measured values obtained from the one or more measuring devices (e.g., 24h-24q) to maintain a selected set-point in the first permeate stream 20a, such as a desired pressure, a desired component composition, and/or a desired flow rate.

For example, the controller 36a may be programmed to acquire one or more process parameters in the first permeate stream 20a using the permeate measuring device 24m. Once a change is detected or a user-specified threshold has been reached, the pressure of the first permeate stream 20a may be adjusted using the first permeate valve 22g to maintain the selected set-point. In one non-limiting example, the composition (e.g., methane concentration) of the first permeate stream 20a is measured and controlled to maintain the selected set-point by adjusting the pressure in the feed gas line 12a using the first gas transport device 14a.

In some instances, the controller 36a may include programming to adjust process parameters in the system 10a using the one or more process control devices (e.g., 14a, 14b, 22e-22k, 28e-28j) in response to measured values obtained from the one or more measuring devices (e.g., 24h-24q) to maintain a selected set-point in the inlet gas line 8a and/or the feed gas line 12a, such as a desired pressure, a desired component composition, and/or a desired flow rate.

In some instances, the controller 36a may include programming to adjust process parameters in the system 10a using the one or more process control devices (e.g., 14a, 14b, 22e-22k, 28e-28j) in response to measured values obtained from the one or more measuring devices (e.g., 24h-24q) to maintain a selected temperature set-point for the membrane separation stage 1a, 2a. For example, the controller 36a may be programmed to acquire one or more process parameters in the system 10a using one or more of the process measuring devices (e.g., 24h-24q) and adjust the operation temperature of the membrane separation stage 1a, 2a using the temperature control device 28f located in the feed gas line 12a, the temperature control device 28g located in the first gas transport adjusted feed stream 16a, and/or the temperature control device 28i located in the first retentate stream 18a to obtain a selected temperature set-point.

It is to be understood that any of the parameters associated with the various gas streams of the system 10a may be determined, measured, altered, and/or adjusted more than once. For example, a first measurement of the one or more values or parameters of a gas stream may be carried out at a first time period, followed by a second measurement carried out at a second time period, where the amount of time that elapses between the first time period and the second time period is determined by the predetermined time interval or another predetermined operational condition. In each instance, such measurements may be carried out by one or more measuring devices (e.g., the one or more process measuring devices 24h-24q) and then received and stored by a controller (e.g., the controller 36a). In addition, a process control system (e.g., the process control system 35a) may take a first action to adjust one or more parameters of a gas stream using one or more process control devices (e.g., the one or more process control devices 14a, 14b, 22e-22k, 28, 28a-28j) at a third time period, followed by a second action carried out at a fourth time period, where the amount of time that elapses between the third time period and the fourth time period is determined by the predetermined time interval or another predetermined operational condition.

Further, in some instances, one or more of the above processes can use machine learning, (ML), artificial intelligence (AI), or similar techniques, to iteratively train the controller 36a and improve the performance of the system based on one or more feedback parameters, characteristics, or similar. For example, in some instances, ML/AI can be used to predict an optimal pressure for the retentate stream 18a based on system data such as pressure, volume, mass, temperature, user preferences, etc. In some instances, ML/AI can be used to determine or predict amounts of a sweep gas to supply from the retentate stream 18a or another source. Thus, the amount of sweep gas returned to the first membrane separation stage 1a in the sweep stream 40a can be optimized. This can be beneficial because the performance and efficiency of the membrane separation stage can be optimized while maintaining or improving one or more outputs of the system 10a.

Referring now to FIG. 3, a system for separating a gas mixture, a system 10b, may be provided in the form of two or more membrane separation stages. Generally, when comparing the system 10b and the systems 10, 10a of FIG. 1 and FIG. 2, respectively, elements having similar names and/or element numbers may have substantially similar structures and functions. Like the system 10, 10a, the system 10b may include an inlet gas line 8b, a gas source 11b, a first gas transport device 14c, a first gas transport adjusted feed stream 16b, a first membrane separation stage 1b, a first retentate stream 18c, a first permeate stream 20c, and a process control system 35b. In addition to the components described with reference to the system 10, 10a, the system 10b may include a second membrane separation stage 2b, a second permeate stream 26, a second retentate stream 29, additional process measuring devices, and/or additional process control devices. The system 10b of FIG. 3 may also include a first membrane separation stage 1b and a second membrane separation stage 2b. In the system 10b, a sweep stream 40b is provided to the permeate side of the second membrane separation stage 2b and integrated with the second membrane separation stage 2b.

In some examples, the system 10b may include at least three membrane separation stages (the additional membrane separation stages are not illustrated). If at least three membrane separation stages are provided in the system 10b, the membrane separation stages may be arranged in series, in parallel, or in series and in parallel. In each instance, at least one membrane separation stage (e.g., the second membrane separation stage 2b) may be coupled to a sweep stream such that a portion of the retentate generated by the membrane separation stage may be provided to the permeate side of said membrane separation stage.

Referring again to FIG. 3, the first permeate stream 20c may be removed as a product, further processed, or discarded. The first retentate stream 18c may be connected to a feed side of the second membrane separation stage 2b. The second membrane separation stage 2b may include a gas separation membrane and a sweep stream 40b, which may be provided to the permeate side of the second membrane separation stage 2b. The second membrane separation stage 2b separates the first retentate stream 18c into a second retentate stream 29 and a second permeate stream 26. The second retentate stream 29 may be withdrawn as a product, further processed, or discarded. In some instances, the second retentate stream 29 is fed or provided to a bio-liquified natural gas (bio-LNG) producing system 30. The obtained bio-LNG may be imparted with a higher purity value than the second retentate stream 29 provided to the bio-LNG producing system 30.

The second permeate stream 26 may be recycled back to the inlet gas line 8b or back to the gas source 11b (recycle lines not shown). Alternatively, the second permeate stream 26 may be further processed, or discarded. In some instances, the second permeate stream 26 is fed or provided to a carbon dioxide recovery system 17b. The carbon dioxide recovery system 17b may prepare purified liquid carbon dioxide from a gas mixture containing carbon dioxide. The obtained purified liquid carbon dioxide may be imparted with a higher purity value than the gas mixture containing carbon dioxide provided to the carbon dioxide recovery system 17b. The carbon dioxide recovery system 17b may compress and cool the gas mixture and, optionally, liquefy the gas mixture (e.g., by using purified liquid carbon dioxide as a cooling medium), whereupon gaseous impurities are either discharged from the liquefied carbon dioxide and vaporized and/or recycled back to the system 10b. In addition, the purified liquid carbon dioxide may be withdrawn as a product. The carbon dioxide recovery system 17b may include at least one compressor (e.g., two compressors), at least one cooler (e.g., two coolers), and purification equipment coupled to the compressor and the cooler via suitable piping (the components of the carbon dioxide recovery system 17b are not illustrated).

In some instances, the controller 36b may adjust or control a multitude of process parameters to maintain a product gas purity. In some non-limiting examples, the product gas in the first retentate stream 18c or second retentate stream 29 comprises mostly methane. For example, the product gas purity of the first retentate stream 18c or second retentate stream 29 may be controlled via the controller 36b to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% methane. In some instances, the product gas purity of the first retentate stream 18c or second retentate stream 29 may be controlled via the controller 36b to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% methane.

In other non-limiting examples, the product gas in the first permeate stream 20c or second permeate stream 26 comprises mostly carbon dioxide. For example, the product gas purity of the first permeate stream 20c or second permeate stream 26 may be controlled via the controller 36b to be at least 80%, or at least 85%, or at least 90%, or at least 91%, or at least 92%, or at least 93%, or at least 94%, or at least 95%, or at least 96%, or at least 97%, or at least 98%, or at least 99%, or at least 99.5%, or at least 99.9% carbon dioxide. As an additional example, the product gas purity of the first permeate stream 20c or second permeate stream 26 may be via the controller 36b to be at least about 80%, or at least about 85%, or at least about 90%, or at least about 91%, or at least about 92%, or at least about 93%, or at least about 94%, or at least about 95%, or at least about 96%, or at least about 97%, or at least about 98%, or at least about 99%, or at least about 99.5%, or at least about 99.9% carbon dioxide.

It is to be understood that the product gas purities described with reference to FIG. 3 may be somewhat less or greater than the values recited herein. In addition, the product gas purities described with reference to FIG. 3 may fall within a range bounded by any minimum and maximum value as described above. Furthermore, the product gas purities described with reference to FIG. 3 may have a value falling within any of the ranges described herein.

Referring still to FIG. 3, in certain instances, about 0.1% to about 15% or 0.1% to 15% of the second retentate stream 29 may be provided to the permeate side of the second membrane separation stage 2b as a sweep gas, although the portion of the second retentate stream 29 utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the second retentate stream 29 may be utilized as a sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 6%, or no more than about 5%, or no more than about 4%, or no more than about 3%, or no more than about 2%, or no more than about 1%, or no more than about 0.5%, or no less than about 0.1% of the second retentate stream 29 may be utilized as a sweep gas.

As yet another example, no more than 15%, or no more than 14%, or no more than 13%, or no more than 12%, or no more than 11%, or no more than 10%, or no more than 9%, or no more than 8%, or no more than 7%, or no more than 6%, or no more than 5%, or no more than 4%, or no more than 3%, or no more than 2%, or no more than 1%, or no more than 0.5%, or no less than 0.1% of the second retentate stream 29 may be utilized as a sweep gas.

In certain instances, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than 15% of the second retentate stream 29 may be utilized as a sweep gas.

In other instances, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the second retentate stream 29 may be utilized as a sweep gas.

It is to be understood that the amount of the second retentate stream 29 utilized as a sweep gas may fall within a range bounded by any minimum and maximum value as described above. Furthermore, the amount of the second retentate stream 29 utilized as a sweep gas may be imparted with a value falling within any of the ranges described herein.

The remaining portion of the second retentate stream 29 may be collected, stored, vented, recycled to the system 10b, and/or further processed.

In some instances, the composition of the sweep stream 40b may be substantially the same as the composition of the second retentate stream 29.

In some instances, the process control system 35b may include a controller 36b, one or more process measuring devices (e.g., 24r-24y), one or more process control devices (e.g., 14c, 22m-22r, 28k-28p), and suitable connections that allow process information acquired by the one or more measuring devices to be transferred to the controller 36b and output information from the controller 36b to be transferred to the one or more process control devices to perform a process control action. Example process control actions may include using the process control devices to alter one or more of a pressure, flow rate, dewpoint, and/or temperature of a process stream based on one or more measured values from the process measuring devices.

Suitable connections may include transmitters that allow process signals, such as electrical signals or gas pressure signals (e.g., air, nitrogen, etc.), to be transmitted between the controller 36b and the process measuring devices (e.g., 24r-24y) and the process control devices (e.g., 14c, 22m-22r, 28k-28p). The electrical signals may be transferred via a wired connection or through a wireless network connection. Other hardware elements may be included in the process control system 35b, for example, transducers, analog-to-digital (A/D) converters, and digital-to-analog (D/A) converters that allow process information to be recognizable in computer form, and computer commands accessible to the process. For visual clarity, the connections between the controller 36b, the one or more measuring devices, and the one or more process control devices have been omitted from FIG. 3.

In some instances, the system 10b may include one or more process measuring devices (e.g., 24r-24y). For example, the system 10b may include one or more sweep stream process measuring devices 24w-24y provided in the sweep stream 40b. Each of the process measuring devices 24r-24y may be configured to measure a process parameter, or multiple process parameters, such as pressure, temperature, flow, composition, viscosity, humidity, moisture, dewpoint, or density.

In some instances, the one or more process measuring devices 24r-24y may include a pressure measuring device. Suitable pressure measuring devices include, but are not limited to, pressure transducers, diaphragm bellows, Bourdon tubes, strain gauges, piezoelectric sensors, ionization gauges, and combinations thereof.

In some instances, the one or more process measuring devices 24r-24y may include a flow measuring device. Suitable flow measuring devices include, but are not limited to, pitot tubes, orifice flow meters, turbine flow meters, electromagnetic field flow meters, neutron bombardment flow meters, ultrasound flow meters, hot-wire anemometry flow meters, and angular momentum flow meters, Coriolis mass flow meters, and combinations thereof.

In some instances, the one or more process measuring devices 24r-24y may include a temperature measuring device. Suitable temperature measuring devices include, but are not limited to, thermocouples, thermistors (e.g., a resistance sensor), oscillating quartz crystal thermometers, radiation pyrometers, and combinations thereof.

In some instances, the one or more process measuring devices 24r-24y may include a density measuring device. Suitable density measuring devices include, but are not limited to, vibrating tube densometers, X-ray densometers, differential pressure densometers, and combinations thereof.

In some instances, the one or more process measuring devices 24r-24y may include a viscosity measuring device. Suitable viscosity measuring devices include, but are not limited to, capillary viscometers, vibrating ball viscometers, and combinations thereof.

In some instances, the one or more process measuring devices 24r-24y may include a humidity measuring device. Suitable humidity measuring devices include hygrometers.

In some instances, the one or more process measuring devices 24r-24y may include a dewpoint measuring device. Suitable dewpoint measuring devices include those available from Vaisala (Finland) and those available from Michell Instruments (e.g., QMA analyzers).

In some instances, the one or more process measuring devices 24r-24y may include a composition measuring device. Suitable composition measuring devices include, but are not limited to, potentiometry sensors, moisture content sensors (hygrometry or psychrometry), gas chromatography, refractive index devices, ultrasound, spectroscopy systems (e.g., UV, visible, IR, Mossbauer, Raman, atomic-emission device, X-ray device, electron, ion, nuclear magnetic resonance systems), polarography devices, conductimetry devices, mass spectrometry systems, differential thermal analysis devices, thermogravimetric analysis systems, and combinations thereof.

Referring still to FIG. 3, the system 10b may include one or more process control devices, such as valves (e.g., 22m-22r), temperature control devices (e.g., 28l-28p), and/or gas transport devices (e.g., 14c). In some instances, the system 10b may include one or more valves 22m-22r that are adjustable between a fully open position and a fully closed position and are used to regulate the pressure of the process streams. For example, the system 10b may include a first sweep stream valve 22p and a second sweep stream valve 22q located in the sweep stream 40b where the first sweep stream valve 22p is upstream of a first sweep stream process measuring device 24w and the second sweep stream valve 22q is downstream of the first sweep stream process measuring device 24w. In some instances, only the first sweep stream valve 22p or only the second sweep stream valve 22q may be provided. In other instances, another valve or an additional valve may be positioned in either the first retentate stream 18c or the sweep stream 40b to control the amount of sweep gas provided to the permeate side of the membrane separation stage 2b. In some examples, the system 10b includes a valve 22r located in the second permeate stream 26. Example valves for use in the system 10b include, without limitation, motor-driven valves, pneumatic valves, and manually adjustable valves.

The system 10b may include one or more process measuring devices (e.g., 24r-24y). For example, the system 10b may include a feed gas line measuring device 24r located in the feed gas line 12b, a measuring device 24s located in the gas transport adjusted feed stream 16b, a first retentate measuring device 24t located in the first retentate stream 18c, a second retentate measuring device 24v located in the second retentate stream 29 upstream of the sweep junction 13b and/or downstream of the sweep junction 13b, a second permeate measuring device 24u located in the second permeate stream 26, and one or more sweep stream process measuring devices 24w, 24x, 24y located in the sweep stream 40b. Each of the process measuring devices 24r-24y may be configured to measure a process parameter, or multiple process parameters, such as pressure, temperature, flow, composition, viscosity, humidity, moisture, dewpoint, or density. In some instances, the one or more sweep stream process measuring devices 24w, 24x, 24y may include a flow meter, a temperature sensor, or both.

The system 10b may include one or more temperature control devices (e.g., 28k-28p) that may be used to adjust the temperature (e.g., heat or cool) of the process streams. For example, the system 10b may include one or more of a temperature control device 28k located in the feed gas line 12b, a temperature control device 28l located in the gas transport adjusted feed stream 16b, a temperature control device 28m located in the first retentate stream 18c, a temperature control device 28n located in the second retentate stream 29, a temperature control device 28p located in the second permeate stream 26, and a sweep stream temperature control device 28o located in the sweep stream 40b. The temperature control device 28o located in the sweep stream 40b may be used to increase the temperature of the sweep stream 40b and prevent the sweep stream 40b, or components within the sweep stream 40b, from undergoing a phase change (e.g., solidifying and/or condensing) from a gas to a non-gas (e.g., liquid or solid) due to the reduction of pressure experienced by the sweep gas in the sweep line 40b. Suitable temperature control devices 28k-28p include devices that heat or cool the process stream, such as heat exchangers or electric heaters.

The controller 36b may include a processor 44b and a memory 48b that includes software 50b and data 52b and is designed for storage and retrieval of processed information to be processed by the processor 44b. The processor 44b may include an input 54b that is configured to receive process signals from the one or more process measuring devices (24r-24y) and from the one or more process control devices (e.g., 14c, 22m-22r, 28k-28p) via the input 54b. The controller 36b may function substantially similarly to the controller 36, 36a. The controller 36b may operate autonomously or semi-autonomously, or it may read executable software instructions from the memory 48b or a computer-readable medium (e.g., a hard drive, a CD-ROM, flash memory), or may receive instructions via the input 54b from a user, or another source logically connected to a computer or device, such as another networked computer or server. For example, the server may be used to control the system 10b via the controller 36b on-site or remotely.

The processor 44b may process the process signals to generate an output 56b, which may take the form of a process control action. Example process control actions may include sending signals to the one or more process control devices (e.g., 14c, 22m-22r, 28k-28p) to effectuate a change in one or more process parameters (e.g., pressure, flow rate, composition, membrane surface area, and/or temperature) of one or more process streams in the system 10b.

In some instances, the controller 36b may include programming to adjust the process parameters in the system 10b using the one or more process control devices (e.g., 14c, 22m-22r, 28k-28p) in response to measured values obtained from the one or more process measuring devices (e.g., 24r-24y) to maintain a selected set-point, such as a desired gas composition (e.g., at least 95% methane) and/or the flow rate of the sweep stream 40b and/or the amount of the second retentate stream 29 provided to the sweep stream 40b.

For example, the controller 36b may be programmed to monitor one or more process parameters (e.g., dewpoint, flow rate) in the sweep stream 40b using the one or more sweep stream process measuring devices 24w, 24x, 24y located in the sweep stream 40b. Once a change in the process parameter is detected or a user-specified threshold has been reached, the pressure of the sweep stream 40b may be adjusted using one or more of the first sweep stream valve 22p and the second sweep stream valve 22q to maintain the selected set-point. The selected set-point in the sweep stream 40b may thus be maintained by adjusting the pressure in the sweep stream 40b using one or more of the sweep stream valves 22p, 22q. Alternatively, or additionally, the feed gas line measuring device 24r may be used to monitor one or more process parameters in the feed gas line 12b, the measuring device 24s located in the gas transport adjusted feed stream 16b may be used to monitor one or more process parameters in the gas transport adjusted feed stream 16b, the first retentate measuring device 24t, located in the first retentate stream 18c, and the second retentate measuring devices 24v, located in the second retentate stream 29, may be used to monitor one or more process parameters in the first retentate stream 18c and second retentate stream 29, respectively.

In some instances, the controller 36b may be in electrical communication with the one or more sweep stream process measuring devices 24w, 24x, 24y and the one or more valves 22p, 22q located in the sweep stream 40b where the controller 36b is configured to maintain or adjust the position of the one or more valves 22p and 22q located in the sweep stream 40b in response to the measured parameter.

In some instances, the controller 36b may include programming to adjust the process parameters in the system 10b using the one or more process control devices (e.g., 14c, 22m-22r, 28k-28p) in response to measured values obtained from the one or more process measuring devices (e.g., 24r-24y) to maintain a selected set-point in the first retentate stream 18c or second retentate stream 29 such as a desired pressure, a desired component composition, and/or a desired flow rate.

For example, the controller 36b may be programmed to monitor one or more process parameters in the first retentate stream 18c or second retentate stream 29 (e.g., pressure, etc.) using the first retentate measuring device 24t located in the first retentate stream 18c. Once a change in the process parameter is detected or a user-specified threshold has been reached, the pressure of the first retentate stream 18c may be adjusted using the first retentate valve 22n to maintain the selected set-point. Alternatively, or additionally, the second retentate measuring device 24v located in the second retentate stream 29 downstream of the sweep junction 13b may be used to monitor one or more process parameters in the second retentate stream 29. Once a change in the process parameter is detected or a user-specified threshold has been reached, the pressure of the second retentate stream 29 downstream of the sweep junction 13b may be adjusted using, for example, another valve (not shown) to maintain the selected set-point.

In some instances, the controller 36b may include programming to adjust process parameters in the system 10b using the one or more process control devices (e.g., 14c, 22m-22r, 28k-28p) in response to measured values obtained from the one or more process measuring devices (e.g., 24r-24y) to maintain a selected set-point in the gas transport adjusted feed stream 16b such as a desired pressure, a desired component composition, and/or a desired flow rate.

In some instances, the controller 36b may include programming to adjust process parameters in the system 10b using the one or more process control devices (e.g., 14c, 22m-22r, 28k-28p) in response to measured values obtained from the one or more measuring devices (e.g., 24r-24y) to maintain a selected set-point in the first permeate stream 20c or second permeate stream 26, such as a desired pressure, a desired component composition, and/or a desired flow rate.

For example, the controller 36b may be programmed to acquire one or more process parameters in the second permeate stream 26 using the permeate measuring device 24u. Once a change is detected or a user-specified threshold has been reached, the pressure of the second permeate stream 26 may be adjusted using the second permeate valve 22r to maintain the selected set-point. In one non-limiting example, the composition (e.g., methane concentration) of the second permeate stream 26 is measured and controlled to maintain the selected set-point by adjusting the pressure in the feed gas line 12b using the first gas transport device 14c.

In some instances, the controller 36b may include programming to adjust process parameters in the system 10b using the one or more process control devices (e.g., 14c, 22m-22r, 28k-28p) in response to measured values obtained from the one or more measuring devices (e.g., 24r-24y) to maintain a selected set-point in the inlet gas line 8b and/or the feed gas line 12b, such as a desired pressure, a desired component composition, and/or a desired flow rate.

In some instances, the controller 36b may include programming to adjust process parameters in the system 10b using the one or more process control devices (e.g., 14c, 22m-22r, 28k-28p) in response to measured values obtained from the one or more measuring devices (e.g., 24r-24y) to maintain a selected temperature set-point for the membrane separation stage 2b. For example, the controller 36b may be programmed to acquire one or more process parameters in the system 10b using one or more of the process measuring devices (e.g., 24r-24y) and adjust the operation temperature of the membrane separation stage 2b using the temperature control device 28k located in the feed gas line 12b, the temperature control device 28l located in the first gas transport adjusted feed stream 16b, and/or the temperature control device 28m located in the first retentate stream 18c to obtain a selected temperature set-point.

It is to be understood that any of the parameters associated with the various gas streams of the system 10b may be determined, measured, altered, and/or adjusted more than once. For example, a first measurement of the one or more values or parameters of a gas stream may be carried out at a first time period, followed by a second measurement carried out at a second time period, where the amount of time that elapses between the first time period and the second time period is determined by the predetermined time interval or another predetermined operational condition. In each instance, such measurements may be carried out by one or more measuring devices (e.g., the one or more process measuring devices 24r-24y) and then received and stored by a controller (e.g., the controller 36b). In addition, a process control system (e.g., the process control system 35b) may take a first action to adjust one or more parameters of a gas stream using one or more process control devices (e.g., the one or more process control devices 14c, 22m-22r, 28k-28p) at a third time period, followed by a second action carried out at a fourth time period, where the amount of time that elapses between the third time period and the fourth time period is determined by the predetermined time interval or another predetermined operational condition.

Further, in some instances, one or more of the above processes can use machine learning, (ML), artificial intelligence (AI), or similar techniques, to iteratively train the controller 36b and improve the performance of the system based on one or more feedback parameters, characteristics, or similar. For example, in some instances, ML/AI can be used to predict an optimal pressure for the second retentate stream 29 based on system data such as pressure, volume, mass, temperature, user preferences, etc. In some instances, ML/AI can be used to determine or predict amounts of a sweep gas to supply from the retentate stream 29 or another source. Thus, the amount of sweep gas returned to the membrane separation stage 2b in the sweep stream 40b can be optimized. This can be beneficial because the performance and efficiency of the membrane separation stage can be optimized while maintaining or improving one or more outputs of the system 10b.

Together, FIGS. 4A and 4B illustrate a schematic diagram of an example process flow to and within a membrane separation stage. The membrane separation stage may be designed to receive a permeate-side sweep gas. Generally, FIG. 4A is used to illustrate the components associated with the example process flow, while FIG. 4B illustrates arrows to depict the various sub-flows within the example process flow. More particularly, the long-dash long-dash arrows may represent the flow of the inlet gas stream provided to the membrane separation stage and the individual membrane modules of the membrane separation stage, the short-dash short-dash arrows may represent the flow of a retentate stream from the individual membrane modules of the membrane separation stage and the membrane separation stage, and the non-dashed arrows may represent the flow of the sweep gas through the membrane separation stage and the individual membrane modules.

The illustrated process flow may be utilized with the membrane separation stages 1, 1a of the systems 10, 10a and/or the membrane separation stage 2b of the system 10b. For ease of reference, the schematic diagram of FIGS. 4A and 4B will be explained with reference to the membrane separation stages 1, 1a of the systems 10, 10a, although a person having skill in the art would appreciate that the teachings of FIG. 4 could also be applied to the membrane separation stage 2b of the system 10b.

The membrane separation stages 1, 1a comprise a plurality of gas separation membrane modules 5a-5n. The plurality of gas separation membrane modules 5a-5n may be arranged in parallel, as shown in FIG. 4, in series, or both in parallel and in series. In some aspects, the membrane separation stage 1, 1a may be configured with a first gas separation membrane module 5a, a second gas separation module 5b, a third gas separation module 5c, and, optionally, one or more additional gas separation modules 5n in fluid communication with the gas stream 59, 59a which is in fluid communication with the gas source 11, 11a. It is to be appreciated that the gas stream 59, 59a may be provided as a stream that is processed or adjusted by a gas transport device, e.g., the gas transport device 14 of FIG. 1. The gas stream 59, 59a may be split into multiple inlet streams 59b-59n, which in turn feed into the plurality of gas separation membrane modules 5a-5n. For example, the incoming stream provided by the gas source 11, 11a may be split into a first interior membrane stage inlet stream 59b, a second interior membrane stage inlet stream 59c, a third interior membrane stage inlet stream 59d, and, optionally, one or more additional interior membrane stage inlet streams 59n. The flow of the gas streams 59-59n is schematically depicted by the long-dash long-dash arrows in FIG. 4B. In some instances, each of the gas separation membrane modules 5a-5n includes a gas separation membrane (e.g., a polymeric hollow fiber membrane) that separates the multiple inlet streams 59b-59n into interior membrane stage permeate streams 58a, 58b, 58c, 58n and interior membrane stage retentate streams 60a, 60b, 60c, 60n, respectively. Each of the interior retentate streams 60a, 60b, 60c, 60n may be combined to form the retentate stream 18, 18a that is provided from the membrane separation stages 1, 1a. The flow of the aforementioned permeate streams is schematically depicted by the short-dash short-dash arrows in FIG. 4B, and the flow of the aforementioned retentate streams is schematically depicted by the long-dash short-dash long-dash arrows in FIG. 4B.

In various instances, feed gas may enter the system through gas stream 59 and may be split into the one or more interior membrane stage inlet streams (e.g., 59b-59n). From the one or more interior membrane stage inlet streams, the feed gas enters the corresponding one or more gas separation membrane modules (e.g., 5a-5n). The one or more interior retentate streams (e.g., 60a-60n) may direct the retentate into the retentate stream 18, which in turn may be in fluid communication with the sweep stream 40 via a sweep junction 13. The retentate in the sweep stream 40 may be either directed out of the system 10 or returns as a sweep gas to the one or more gas separation membrane modules via the interior sweep streams (e.g. 70a-70n). The flow of the sweep gas is schematically depicted by the solid (i.e., non-dashed) arrows in FIG. 4B. Permeate may exit the one or more gas separation membrane modules via the interior permeate streams (e.g. 58a-58n). The interior permeate streams may be in fluid communication with the permeate stream 20, such that permeate may pass through the interior permeate streams into the permeate stream 20, where the permeate may be directed out of the system 10. In some cases, the system described may comprise one or more valves that may be designed to control the volume of flow through each of the above described streams, such that the volume of flow through the streams may be increased, decreased, or ceased.

The membrane modules 5a-5n may be provided in the form of a thin film that is manufactured from polymers, metals, or ceramics. In some instances, the one or more gas separation membrane modules comprise polymeric membranes, ceramic membranes, metal membranes, or a combination thereof. For example, the one or more gas separation membrane modules may comprise mixed-matrix membranes formed by incorporating an inorganic filler (e.g., a zeolite) into a continuous polymeric matrix (e.g., a polyimide). In other instances, the one or more gas separation membrane modules comprise polymeric membranes. For example, the one or more gas separation membranes used to construct the membrane modules may comprise polyimides, polycarbonates, polyphenyl oxide, polysulfones, cellulose derivatives, polyethylene oxide, and/or combinations thereof. In other instances, the one or more gas separation membrane modules comprise inorganic membranes. For example, the one or more gas separation membrane modules may comprise zeolite membranes. The membrane material may be chosen based on the selectivity of the material for one or more gases in the gas mixture. As an additional example, the one or more gas separation membrane modules may comprise polyimide hollow fiber membranes.

In some instances, interior sweep streams 70a-70n are provided to the permeate sides of the gas separation membrane modules 5a-5n. The interior sweep streams 70a-70n may be provided as a portion of the retentate stream 18, 18a that is generated from the interior retentate streams 60a, 60b, 60c, 60n. For example, the sweep stream 40, 40a may be split into a first interior sweep stream 70a, a second interior sweep stream 70b, a third interior sweep stream 70c, and, optionally, one or more additional interior membrane stage sweep streams 70n. The retentate stream 18, 18a flows to a sweep junction 13, 13a, where a selected portion of the CH₄-rich retentate gas is separated from the retentate stream 18, 18a and allocated to the interior sweep streams 70a-70n.

The amount of retentate gas allocated to the interior sweep streams 70a-70n may range from 0.1% to 15% of the retentate stream 18, 18a. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the second retentate stream 29 may be utilized as a sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 6%, or no more than about 5%, or no more than about 4%, or no more than about 3%, or no more than about 2%, or no more than about 1%, or no more than about 0.5%, or no less than about 0.1% of the retentate stream 18, 18a may be provided to the interior sweep streams 70a-70n.

As yet another example, no more than 15%, or no more than 14%, or no more than 13%, or no more than 12%, or no more than 11%, or no more than 10%, or no more than 9%, or no more than 8%, or no more than 7%, or no more than 6%, or no more than 5%, or no more than 4%, or no more than 3%, or no more than 2%, or no more than 1%, or no more than 0.5%, or no less than 0.1% of the retentate stream 18, 18a may be provided to the interior sweep streams 70a-70n.

In certain instances, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than 15% of the retentate stream 18, 18a may be provided to the interior sweep streams 70a-70n.

In other instances, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the retentate stream 18, 18a may be provided to the interior sweep streams 70a-70n.

In certain instances, the amount of the sweep gas allocated to the interior sweep streams 70a-70n (i.e., the amount of the retentate stream 18, 18a provided to the interior sweep streams 70a-70n) can be defined relative to the amount of gas provided to the feed gas line 12, 12a (see FIGS. 1 and 2). For example, the amount of sweep gas can be represented as a percentage of the overall volume or mass of feed gas supplied to the system 10, 10a. In some such instances, 0.1% to 8% of the feed gas provided to the feed gas line 12, 12a may ultimately be allocated to the interior sweep streams 70a-70n, although the amount of the feed gas utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8% may be utilized as a sweep gas. As an additional example, no more than 8%, or no more than 7%, or no more than 6%, or no more than 5%, or no more than 4.5%, or no more than 4%, or no more than 3.5%, or no more than 3%, or no more than 2%, or no more than 2.5%, or no more than 1%, or no more than 0.5%, or no less than 0.1% of the feed gas provided to the feed gas line 12a may be allocated to the interior sweep streams 70a-70n.

In other such instances, about 0.1% to about 8% of the feed gas provided to the feed gas line 12, 12a may be utilized as a sweep gas, although the amount of the feed gas utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8% of the feed gas provided to the feed gas line 12, 12a may be utilized as a sweep gas. As an additional example, no more than about 8%, or no more than about 7%, or no more than about 6%, or no more than about 5%, or no more than about 4.5%, or no more than about 4%, or no more than about 3.5%, or no more than about 3%, or no more than about 2.5%, or no more than about 2%, or no more than about 1.5%, or no more than about 1%, or no more than about 0.5%, or no less than about 0.1% of the feed gas provided to the feed gas line 12a may be allocated to the interior sweep streams 70a-70n.

In certain instances, at least about 0.1%, or at least about 0.5%, or at least about 1%, or at least about 1.5%, or at least about 2%, or at least about 2.5%, or at least about 3%, or at least about 3.5%, or at least about 4%, or at least about 4.5%, or at least about 5%, or at least about 6%, or at least about 7%, or no more than 8% of the feed gas provided to the feed gas line 12a may be allocated to the interior sweep streams 70a-70n.

In other instances, about 0.1%, or about 0.5%, or about 1%, or about 1.5%, or about 2%, or about 2.5%, or about 3%, or about 3.5%, or about 4%, or about 4.5%, or about 5%, or about 6%, or about 7%, or about 8% of the feed gas provided to the feed gas line 12a may be allocated to the interior sweep streams 70a-70n.

The amount of retentate gas allocated to the interior sweep streams 70a-70n may be adjusted or controlled by one or more process control devices (e.g., the process control devices 22, 22a, 22e, 22f). In the illustrated instance of FIG. 4, only one process control device (i.e., the process control device 22a, 22f) is provided, although it is understood that additional process control devices may also be provided in the sweep stream 40, 40a, or in any of the interior sweep streams 70a-70n.

### Methods of Separating a Gas Mixture

A method of separating a gas mixture is provided. In some instances, the gas mixture comprises biogas or natural gas. The method includes providing a gas mixture comprising at least methane and carbon dioxide; feeding the gas mixture to a membrane separation stage comprising at least one gas separation membrane module configured to separate the gas mixture into a retentate stream and a permeate stream, wherein the at least one gas separation membrane module comprises a membrane having a permeate side and a retentate side, wherein the pressure of gas on the retentate side is greater than the pressure of gas on the permeate side; and feeding a sweep stream along the permeate side of the membrane, wherein the sweep stream comprises a portion of the retentate stream.

In some instances of the above-described method, the retentate stream comprises primarily methane and the permeate stream comprises primarily carbon dioxide.

In some instances of the above-described method, 0.1% to 15% of the retentate stream is provided to the permeate side of the membrane separation stage as a sweep gas, although the portion of the retentate stream utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the retentate stream may be utilized as a sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 6%, or no more than about 5%, or no more than about 4%, or no more than about 3%, or no more than about 2%, or no more than about 1%, or no more than about 0.5%, or no less than about 0.1% of the retentate stream may be utilized as a sweep gas.

As yet another example, no more than 15%, or no more than 14%, or no more than 13%, or no more than 12%, or no more than 11%, or no more than 10%, or no more than 9%, or no more than 8%, or no more than 7%, or no more than 6%, or no more than 5%, or no more than 4%, or no more than 3%, or no more than 2%, or no more than 1%, or no more than 0.5%, or no less than 0.1% of the retentate stream may be utilized as a sweep gas.

In certain instances, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than 15% of the retentate stream may be utilized as a sweep gas.

In other instances, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the retentate stream may be utilized as a sweep gas.

It is to be understood that the amount of the retentate stream utilized as a sweep gas may fall within a range bounded by any minimum and maximum value as described above. Furthermore, the amount of the retentate stream utilized as a sweep gas may be imparted with a value falling within any of the ranges described herein.

In certain instances, the amount of the sweep gas provided to the permeate side of the membrane separation stage (i.e., the amount of the retentate stream provided to the membrane separation stage) can be defined relative to the amount of gas provided to the feed stream line. For example, the amount of sweep gas can be represented as a percentage of the overall volume or mass of feed gas supplied to the system. In some such instances, 0.1% to 8% of the feed gas provided to the feed stream line may ultimately be utilized as a sweep gas, although the amount of the feed gas utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8% may be utilized as a sweep gas. As an additional example, no more than 8%, or no more than 7%, or no more than 6%, or no more than 5%, or no more than 4.5%, or no more than 4%, or no more than 3.5%, or no more than 3%, or no more than 2.5% or no more than 2%, or no more than 1.5%, or no more than 1%, or no more than 0.5%, or no less than 0.1% of the feed gas provided to the feed gas line may be utilized as a sweep gas.

In other such instances, about 0.1% to about 8% of the feed gas provided to the feed gas line 12 may be utilized as a sweep gas, although the amount of the feed gas utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8% of the feed gas provided to the feed gas line may be utilized as a sweep gas.

As an additional example, at least about 8%, or at least about 7%, or at least about 6%, or at least about 5%, or at least about 4.5%, or at least about 4%, or at least about 3.5%, or at least about 3%, or at least about 2.5%, or at least about 2%, or at least about 1.5%, or at least about 1%, or at least about 0.5%, or at least about 0.1% of the feed gas provided to the feed gas line may be utilized as a sweep gas.

In other instances, about 0.1%, or about 0.5%, or about 1%, or about 1.5%, or about 2%, or about 2.5%, or about 3%, or about 3.5%, or about 4%, or about 4.5%, or about 5%, or about 6%, or about 8% of the feed gas provided to the feed gas line may be utilized as a sweep gas.

It is to be understood that the amount of the feed gas utilized as a sweep gas may fall within a range bounded by any minimum and maximum value as described above. Furthermore, the amount of the feed gas utilized as a sweep gas may be imparted with a value falling within any of the ranges described herein.

In some instances of the above-described method, the method may also include a step of providing a process control system comprising a controller, one or more process measuring devices, and one or more process control devices.

In some instances of the above-described method, the method further may comprise a step of providing a controller in electrical communication with one or more measuring devices configured to measure a parameter of the sweep stream, the retentate stream, and/or the permeate stream.

In some instances of the above-described method, the method may comprise a step of providing a controller in electrical communication with one or more process control devices configured to adjust or alter at least one parameter of the sweep stream, the retentate stream, and/or the permeate stream. In some such instances, the controller may adjust a process control device of the one or more process control devices if a parameter of the least one parameter of the sweep stream, the retentate stream, and/or the permeate stream is above or below a predetermined threshold value (e.g., a predetermined set-point value).

Another method of separating a gas mixture is provided. In some instances, the gas mixture comprises biogas or natural gas. The method may include providing a gas mixture comprising methane and carbon dioxide; feeding the gas mixture to a first membrane separation stage comprising at least one gas separation membrane module configured to separate the gas mixture into a first retentate stream and a first permeate stream; providing a sweep stream to the permeate side of the membrane of the at least one gas separation membrane module of the first membrane separation stage, wherein the sweep stream comprises a portion of the first retentate stream; and feeding the first permeate stream to a second membrane separation stage comprising at least one gas separation membrane module configured to separate the first permeate stream into a second retentate stream and a second permeate stream, wherein the at least one gas separation membrane module of the second membrane separation stage comprises a membrane having a permeate side and a retentate side.

In some instances of the above-described method, the retentate stream comprises primarily methane and the permeate stream comprises primarily carbon dioxide.

In some instances of the above-described method, 0.1% to 15% of the retentate stream is provided to the permeate side of the membrane separation stage as a sweep gas, although the portion of the retentate stream utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8%, or about 8% to about 9%, or about 9% to about 10%, or about 10% to about 11%, or about 11% to about 12%, or about 12% to about 13%, or about 13% to about 14%, or about 14% to about 15% of the second retentate stream may be utilized as a sweep gas. As an additional example, no more than about 15%, or no more than about 14%, or no more than about 13%, or no more than about 12%, or no more than about 11%, or no more than about 10%, or no more than about 9%, or no more than about 8%, or no more than about 7%, or no more than about 6%, or no more than about 5%, or no more than about 4%, or no more than about 3%, or no more than about 2%, or no more than about 1%, or no more than about 0.5%, or no less than about 0.1% of the second retentate stream may be utilized as a sweep gas.

As yet another example, no more than 15%, or no more than 14%, or no more than 13%, or no more than 12%, or no more than 11%, or no more than 10%, or no more than 9%, or no more than 8%, or no more than 7%, or no more than 6%, or no more than 5%, or no more than 4%, or no more than 3%, or no more than 2%, or no more than 1%, or no more than 0.5%, or no less than 0.1% of the second retentate stream may be utilized as a sweep gas.

In certain instances, at least about 0.1%, or at least about 0.5%, or at least about 1%, or at least about 2%, or at least about 3%, or at least about 4%, or at least about 5%, or at least about 6%, or at least about 7%, or at least about 8%, or at least about 9%, or at least about 10%, or at least about 11%, or at least about 12%, or at least about 13%, or at least about 14%, or no more than 15% of the second retentate stream may be utilized as a sweep gas.

In other instances, about 0.1%, or about 0.5%, or about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the second retentate stream may be utilized as a sweep gas.

In some such instances, the flow rate of the sweep stream may range from 3 Nm³/h to 4 Nm³/h, although the flow rate of the sweep stream may also be less than or greater than these values. For example, the flow rate of the sweep stream may range from 3.2 Nm³/h to 3.8 Nm³/h, or 3.4 Nm³/h to 3.6 Nm³/h.

In certain instances, the amount of the sweep gas provided to the permeate side of the membrane separation stage (i.e., the amount of the retentate stream provided to the membrane separation stage) can be defined relative to the amount of gas provided to the feed stream line. For example, the amount of sweep gas can be represented as a percentage of the overall volume or mass of feed gas supplied to the system 10. In some such instances, 0.1% to 8% of the feed gas provided to the feed stream line may ultimately be utilized as a sweep gas, although the amount of the feed gas utilized as a sweep gas may be somewhat less or somewhat greater than these values. For example, 0.1% to 0.5%, or 0.5% to 1%, or 1% to 2%, or 2% to 3%, or 4% to 5%, or 5% to 6%, or 6% to 7%, or 7% to 8% may be utilized as a sweep gas. As an additional example, no more than 8%, or no more than 7%, or no more than 6%, or no more than 5%, or no more than 4.5%, or no more than 4%, or no more than 3.5%, or no more than 3%, or no more than 2%, or no more than 2.5%, or no more than 1%, or no more than 0.5%, or no less than 0.1% of the feed gas provided to the feed gas line may be utilized as a sweep gas.

In other such instances, about 0.1% to about 8% of the feed gas provided to the feed gas line 12 may be utilized as a sweep gas, although the amount of the feed gas utilized as a sweep gas may be somewhat less or somewhat greater than these values. For example, about 0.1% to about 0.5%, or about 0.5% to about 1%, or about 1% to about 2%, or about 2% to about 3%, or about 3% to about 4%, or about 4% to about 5%, or about 5% to about 6%, or about 6% to about 7%, or about 7% to about 8% of the feed gas provided to the feed gas line 12 may be utilized as a sweep gas. As an additional example, no more than about 8%, or no more than about 7%, or no more than about 6%, or no more than about 5%, or no more than about 4.5%, or no more than about 4%, or no more than about 3.5%, or no more than about 3%, or no more than about 2.5%, or no more than about 2%, or no more than about 1.5%, or no more than about 1%, or no more than about 0.5%, or no less than about 0.1% of the feed gas provided to the feed gas line may be utilized as a sweep gas.

In certain instances, at least about 0.1%, or at least about 0.5%, or at least about 1%, or at least about 1.5%, or at least about 2%, or at least about 2.5%, or at least about 3%, or at least about 3.5%, or at least about 4%, or at least about 4.5%, or at least about 5%, or at least about 6%, or at least about 7%, or at least about 8% of the feed gas provided to the feed gas line may be utilized as a sweep gas.

In other instances, about 0.1%, or about 0.5%, or about 1%, or about 1.5%, or about 2%, or about 2.5%, or about 3%, or about 3.5%, or about 4%, or about 4.5%, or about 5%, or about 6%, or about 7%, or about 8% of the feed gas provided to the feed gas line may be utilized as a sweep gas.

In some instances of the above-described method, the method further comprises a step of providing a controller in electrical communication with one or more measuring devices configured to measure a parameter of the sweep stream, the first retentate stream, the second retentate stream, the first permeate stream, and/or the second permeate stream.

In further instances, the method further comprises comparing a value of the parameter measure by the one or more measuring devices with a set point value of the parameter. If the difference in the value of the parameter and the set point value of the parameter is determined to be outside of a predetermined acceptable range, the method may comprise a further step of adjusting one or more valves in fluid communication with at least one of the sweep stream, the retentate stream, and the permeate stream. The adjustment of the valve may be designed to increase the flow, decrease the flow, or cease the flow of fluid through the valve.

In some instances of the above-described method, the method may also include a step of providing a process control system comprising a controller, one or more process measuring devices, and one or more process control devices.

In some instances of the above-described method, the method further may comprise a step of providing a controller in electrical communication with one or more measuring devices configured to measure a parameter of the sweep stream, a retentate stream, and/or a permeate stream.

In some instances of the above-described method, the method may comprise a step of providing a controller in electrical communication with one or more process control devices configured to adjust or alter at least one parameter of the sweep stream, a retentate stream, and/or a permeate stream. In some such instances, the controller may adjust a process control device of the one or more process control devices if a parameter of the least one parameter of the sweep stream, the retentate stream, and/or the permeate stream is above or below a predetermined threshold value (e.g., a predetermined set-point value).

The gas separation system of the above-described methods may be any gas separation system 10, 10a, 10b, and any variations thereof, disclosed herein. Thus, the above-described methods may utilize any and all of the components of the gas separation system 10, 10a, 10b described herein, as desired.

It is to be appreciated that the above-described methods may include or omit any of the steps described herein. In addition, the steps of the above-described method may be performed in any order. Furthermore, the above-described methods may include additional steps consistent with the teachings herein.

### Examples

### Example 1

Table 1 illustrates process data for an example process of separating methane from biogas using system 10, as depicted in FIG. 1. The test simulation data is generated with a system as depicted in FIG. 1, namely including a sweep stream 40. The comparative simulation data is generated with a system similar to the system of FIG. 1 but without the sweep stream in the first membrane separation stage. The test simulation system and the comparative simulation system are run under similar conditions.

**Table 1**

| | Comparative | Test |
|---|---|---|
| CH₄ Loss | 0% | 0% |
| # of Modules | 21 | 13s* |
| Relative Specific Power Consumption | 1 | 1.02 |

| | | |
|---|---|---|
| *A number followed by the letter "s" refers to the number of sweep modules (modules with internal sweeps). A number without a letter "s" refers to a standard module without an internal sweep. | | |

As shown in Table 1, the test simulation system utilized fewer sweep modules and, thereby, less membrane surface area. The comparative simulation data utilized a greater number of standard modules. At the same time, the power consumption of the test simulation system is approximately the same as the power consumption of the comparative simulation system. Altogether, the use of fewer membrane modules makes the test simulation system more efficient at a relatively similar power consumption.

### Example 2

Table 2 illustrates process data for an example process of separating methane from biogas using system 10a, as depicted in FIG. 2. The test simulation data is generated with a system as depicted in FIG. 2, namely including a sweep stream 40a at the first membrane separation stage 1a. The comparative simulation data is generated with a system similar to the system of FIG. 2 but without the sweep stream. The test simulation system and the comparative simulation system are run under similar conditions.

**Table 2**

| | Comparative | Test |
|---|---|---|
| CH₄ Loss | 0% | 0% |
| # of Membrane Modules | 21 | 12s+4 |
| Relative Specific Power Consumption | 1 | 0.96 |

| | | |
|---|---|---|
| *A number followed by the letter "s" refers to the number of sweep modules (modules with internal sweeps). A number without a letter "s" refers to a standard module without an internal sweep. | | |

As shown in Table 2, the test simulation system utilized fewer sweep modules and, thereby, less membrane surface area. At the same time, the power consumption of the test simulation system is approximately the same as the power consumption of the comparative simulation system. Altogether, the use of fewer membrane modules and a lower specific power consumption makes the test simulation system more efficient.

### Example 3

Table 3 illustrates process data for an example process of separating methane from biogas using system 10a, as depicted in FIG. 2. The test simulation data is generated with a system as depicted in FIG. 2, namely including a sweep stream 40a at the first membrane separation stage 1a. The comparative simulation data is generated with a system comprising a three-stage membrane separation system without a sweep stream (not shown), where the feed of the second stage is in fluid communication with the retentate of the first stage and the feed of the third stage is in fluid communication with the permeate of the first stage. The test simulation system and the comparative simulation system are run under similar conditions.

**Table 3**

| | Comparative | Test |
|---|---|---|
| CH₄ Loss | 0.5% | 0.5% |
| # of Membrane Modules | 36 | 12s + 4 |

| | | |
|---|---|---|
| *A number followed by the letter "s" refers to the number of sweep modules (modules with internal sweeps). A number without a letter "s" refers to a standard module without an internal sweep. | | |

As shown in Table 3, the test simulation system utilized fewer membrane modules and, thereby, less membrane surface area. At the same time, the test simulation system exhibits a lower compressor flow rate than the comparative simulation system, indicating that a smaller capacity compressor may be utilized in the test simulation system. Altogether, the use of fewer membrane modules and a lower compressor flow rate makes the test simulation system more efficient. In addition, in the test simulation of Example 3, a CO₂ liquefaction unit(s) may be added to convert the second stage permeate into liquified CO₂.

### Example 4

Table 4 illustrates process data for an example process of producing bio-liquified natural gas (LNG) from biogas as depicted in FIG. 3. The test simulation data is generated with a system as depicted in FIG. 3, namely including a sweep stream 40 that is integrated with the second membrane separation stage 2. The comparative simulation data is generated with a system comprising a two-stage membrane separation system with a separate (non-integrated) sweep stage (not shown). The test simulation system and the comparative simulation system are run under similar conditions.

**Table 4**

| | Comparative | Test |
|---|---|---|
| CH₄ Loss | 0% | 0% |
| # of Membrane Modules | 21 | 19 |

As shown in Tables 1, 2, 3, and 4, the systems described herein provide significant advantages, including the removal of excess CO₂ with the addition of a first-stage sweep stream. This increases the efficiency and cost-effectiveness of the system compared to the prior art.

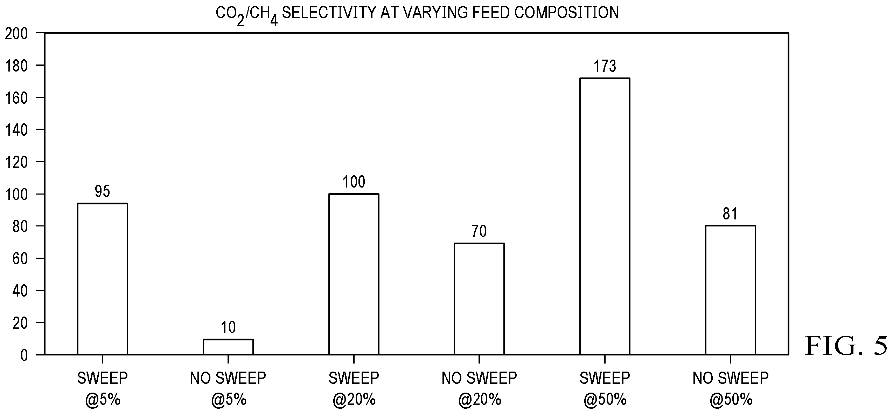

Referring now to FIG. 5, the illustrated bar graph shows CO₂/CH₄ selectivity as a function of the inclusion of a sweep stream in the system and with various sweep stream compositions, in which the inclusion of a sweep stream and its composition are shown on the x-axis and the CO₂/CH₄ selectivities are shown on the y-axis. The first two bars closest to the y-axis respectively represent a system with a feed gas containing 5% (normalized v/v) CO₂ and a sweep stream and the same system without a sweep stream. The middle two bars respectively represent a system with a feed gas containing 20% (normalized v/v) CO₂ and a sweep stream and the same system without a sweep stream. The last two bars respectively represent a system with a feed gas containing 50% (normalized v/v) CO₂ and a sweep stream and the same system without a sweep stream. During the test, the sweep was fed internally and kept constant at 3-4 Nm³/h.

As shown in FIG. 5, the inclusion of a sweep stream consistently increases CO₂/CH₄ selectivity, regardless of the stream composition. FIG. 5 also shows that the composition of the feed stream and consequently the sweep stream affects CO₂/CH₄ selectivity: as the concentration of CH₄ in the sweep stream increases, the CO₂/CH₄ selectivity increases. Without being bound to a particular theory, it is believed that when the permeate side is swept with substantially pure or purified CH₄, the CO₂ concentration at the membrane surface on the permeate side is forced to go into such a low partial pressure regime such that its solubility becomes high due to the dual sorption mode. This enhanced solubility at this permeate membrane interface results in competitive sorption that is strongly favored towards CO₂, resulting in increased CO₂ permeability across the membrane and consequently selectivity. This is also supported by the higher efficacy of the sweep stream at lower CO₂ fractions in the feed.

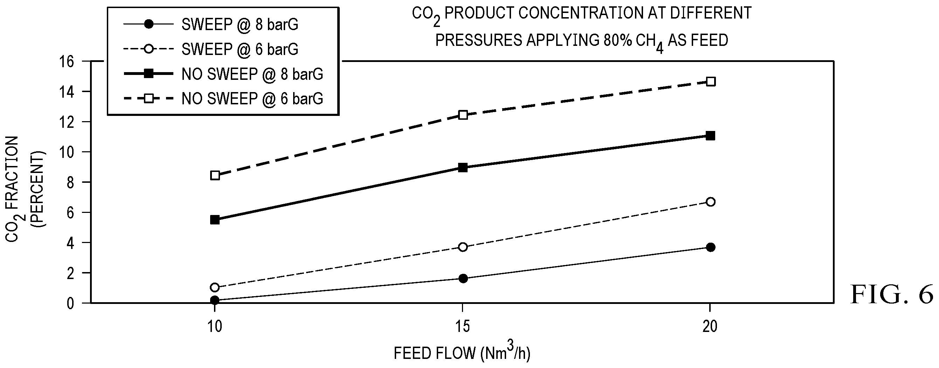

FIG. 6 shows a line graph illustrating the relationship between a gas feed flow rate and the CO₂ concentration of the retentate, at 6 barG and 8 barG of feed pressure and with a feed gas comprising 80% CH₄. During the test, the sweep was fed internally (from a retentate stream) and kept constant at 3-4 Nm³/h.

As shown in FIG. 6, the inclusion of a sweep stream consistently yields a decreased concentration of CO₂ in the product gas, regardless of the feed pressure. The concentration of CO₂ in the product gas is even less in the system that includes a sweep stream at the higher feed pressure (8 barG) than the system that includes the sweep stream at the lower feed pressure (6 barG). It is believed that a higher feed pressure yields a more CH₄-rich/CO₂-lean retentate and consequently a more CH₄-rich/CO₂-lean sweep stream. Also, as the feed flow increases, the concentration of CO₂ in the retentate increases, regardless of the inclusion of a sweep stream or the feed pressure.

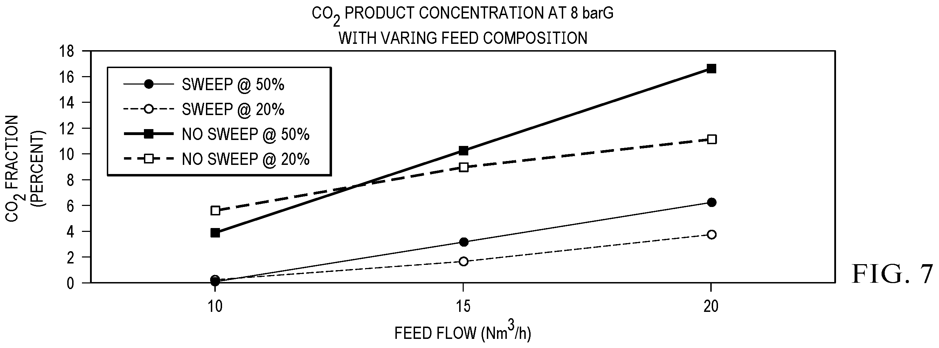

FIG. 7 shows a line graph illustrating the relationship between gas feed flow rate and the CO₂ concentration of the product gas, at 8 barG of feed pressure, with or without the inclusion of a sweep stream in the system and using various compositions of feed gas.

As shown in FIG. 7, the inclusion of a sweep stream consistently yields a decreased concentration of CO₂ in the product gas, regardless of the composition of the feed gas. Also, at increased feed flows, the use of a sweep stream is even more effective for more CO₂-rich feed gas compositions.

Additional aspects of the disclosure will be shown by way of example:
In Example 1, a system for separating a gas mixture, the system comprising: a source of the gas mixture comprising methane and carbon dioxide; a first membrane separation stage in fluid communication with the source of the gas mixture, the first membrane separation stage comprising a gas separation membrane module configured to separate the gas mixture into a retentate stream and a permeate stream, wherein the gas separation membrane module includes a membrane having a permeate side and a retentate side; and a sweep stream provided to the permeate side of the membrane, wherein the sweep stream comprises a portion of the retentate stream.

In Example 2, the system of Example 1, the system further comprising a controller in electrical communication with one or more process measuring devices configured to measure at least one parameter of the sweep stream, the retentate stream, or the permeate stream.

In Example 3, the system of Example 2, the system further comprising one or more process control devices in fluid communication with at least one of the sweep stream, the retentate stream, and the permeate stream, wherein the controller is configured to send a signal to the one or more process control devices in response to a measurement of the at least one parameter.

In Example 4, the system of Example 2 or 3, wherein the at least one parameter is selected from the group consisting of pressure, temperature, flow rate, composition, viscosity, humidity, moisture content, dewpoint, density, and combinations thereof.

In Example 5, the system of any one of Examples 2 to 4, wherein the controller compares a value of the at least one parameter measured by the one or more process measuring devices with a set-point value of the at least one parameter.

In Example 6, the system of any one of Examples 2 to 5, the system further comprising one or more valves in fluid communication with at least one of the sweep stream, the retentate stream, and the permeate stream, wherein the controller is configured to maintain or adjust a position of the one or more valves in response to a measurement of the at least one parameter.

In Example 7, the system of any one of Examples 1 to 6, the system further comprising at least one gas transport device with an inlet in fluid communication with the gas mixture and an outlet in fluid communication with the gas separation membrane module.

In Example 8, the system of any one of Examples 1 to 7, wherein about 0.1% to about 15% of the retentate stream is provided to the permeate side of the gas separation membrane module as a sweep gas.

In Example 9, a system for separating a gas mixture, the system comprising: a first membrane separation stage comprising at least one gas separation membrane module configured to separate the gas mixture into a first retentate stream and a first permeate stream, wherein the gas mixture comprises methane and carbon dioxide; a sweep stream in fluid communication with the first membrane separation stage, the sweep stream provided to a permeate side of the first membrane separation stage, wherein the sweep stream comprises a gaseous component; and a second membrane separation stage configured to separate the first permeate stream into a second retentate stream and a second permeate stream.

In Example 10, the system of Example 9, the system further comprising: a first gas transport device with a first inlet in fluid communication with the gas mixture and a first outlet in fluid communication with the first membrane separation stage; and a second gas transport device with a second inlet in fluid communication with the first permeate stream and a second outlet in fluid communication with the second membrane separation stage.

In Example 11, the system of Example 9 or 10, wherein the first membrane separation stage comprises a first plurality of membranes having a first total membrane surface area.

In Example 12, the system of any one of Examples 9 to 11, wherein the second membrane separation stage comprises a second plurality of membranes having a second total membrane surface area.

In Example 13, the system of any one of Examples 9 to 12, wherein the sweep stream comprises at least a portion of the first retentate stream.

In Example 14, the system of any one of Examples 9 to 13, wherein the sweep stream comprises a gas stream sourced from outside of the system.

In Example 15, the system of any one of Examples 9 to 14, wherein the gas mixture is provided as a feed gas to the system, and the sweep stream comprises no more than about 8% of the feed gas.

In Example 16, a method for separating a gas mixture, the method comprising: providing the gas mixture; feeding the gas mixture to a first membrane separation stage comprising at least one gas separation membrane module configured to separate the gas mixture into a first retentate stream and a first permeate stream; and providing a sweep stream to a permeate side of the at least one gas separation membrane module of the first membrane separation stage, wherein the sweep stream comprises a portion of the first retentate stream.

In Example 17, the method of Example 16, the method further comprising feeding the first permeate stream to a second membrane separation stage comprising at least one gas separation membrane module configured to separate the first permeate stream into a second retentate stream and a second permeate stream.

In Example 18, the method of Example 17, the method further comprising providing a controller in electrical communication with one or more measuring devices configured to measure at least one parameter of the sweep stream, the first retentate stream, the second retentate stream, the first permeate stream, or the second permeate stream.

In Example 19, the method of Example 18, the method further comprising: determining a value of the at least one parameter; comparing the value of the at least one parameter to a set-point value via the controller; and adjusting a valve in fluid communication with the sweep stream if the value of the at least one parameter is above or below a predetermined threshold value.

In Example 20, the method of any one of Examples 16 to 19, wherein the sweep stream comprises no more than about 15% of the first retentate stream and the sweep stream is at least about 80% methane.

It will be appreciated by those skilled in the art that while the invention has been described above in connection with particular instances and examples, the invention is not necessarily so limited, and that numerous other instances, examples, uses, modifications and departures from the instances, examples and uses are intended to be encompassed by the claims attached hereto. The entire disclosure of each patent and publication cited herein is incorporated by reference, as if each such patent or publication were individually incorporated by reference herein. Various features and advantages of the invention are set forth in the following claims.

## Claims

1. A system for separating a gas mixture, the system comprising:
a source of the gas mixture comprising methane and carbon dioxide;
a first membrane separation stage in fluid communication with the source of the gas mixture, the first membrane separation stage comprising a gas separation membrane module configured to separate the gas mixture into a retentate stream and a permeate stream, wherein the gas separation membrane module includes a membrane having a permeate side and a retentate side; and
a sweep stream provided to the permeate side of the membrane, wherein the sweep stream comprises a portion of the retentate stream.

2. The system of claim 1, the system further comprising a controller in electrical communication with one or more process measuring devices configured to measure at least one parameter of the sweep stream, the retentate stream, or the permeate stream.

3. The system of claim 2, the system further comprising one or more process control devices in fluid communication with at least one of the sweep stream, the retentate stream, and the permeate stream, wherein the controller is configured to send a signal to the one or more process control devices in response to a measurement of the at least one parameter.

4. The system of claim 2 or 3, wherein the at least one parameter is selected from the group consisting of pressure, temperature, flow rate, composition, viscosity, humidity, moisture content, dewpoint, density, and combinations thereof.

5. The system of any one of claims 2 to 4, wherein the controller compares a value of the at least one parameter measured by the one or more process measuring devices with a set-point value of the at least one parameter.

6. The system of any one of claims 2 to 5, the system further comprising one or more valves in fluid communication with at least one of the sweep stream, the retentate stream, and the permeate stream, wherein the controller is configured to maintain or adjust a position of the one or more valves in response to a measurement of the at least one parameter.

7. The system of any one of claims 1 to 6, the system further comprising at least one gas transport device with an inlet in fluid communication with the gas mixture and an outlet in fluid communication with the gas separation membrane module.

8. The system of any one of claims 1 to 7, wherein about 0.1% to about 15% of the retentate stream is provided to the permeate side of the gas separation membrane module as a sweep gas.

9. A method for separating a gas mixture, the method comprising:
providing the gas mixture;
feeding the gas mixture to a first membrane separation stage comprising at least one gas separation membrane module configured to separate the gas mixture into a first retentate stream and a first permeate stream; and
providing a sweep stream to a permeate side of the at least one gas separation membrane module of the first membrane separation stage, wherein the sweep stream comprises a portion of the first retentate stream.

10. The method of claim 9, the method further comprising feeding the first permeate stream to a second membrane separation stage comprising at least one gas separation membrane module configured to separate the first permeate stream into a second retentate stream and a second permeate stream.

11. The method of claim 10, the method further comprising providing a controller in electrical communication with one or more measuring devices configured to measure at least one parameter of the sweep stream, the first retentate stream, the second retentate stream, the first permeate stream, or the second permeate stream.

12. The method of claim 11, the method further comprising:
determining a value of the at least one parameter;
comparing the value of the at least one parameter to a set-point value via the controller; and
adjusting a valve in fluid communication with the sweep stream if the value of the at least one parameter is above or below a predetermined threshold value.

13. The method of any one of claims 9 to 12, wherein the sweep stream comprises no more than about 15% of the first retentate stream and the sweep stream is at least about 80% methane.
